# EUROPEAN PATENT APPLICATION

(11) **EP 2 301 627 A1**
(43) Date of publication of application: **30.03.2011**
(21) Application number: 09450178.0
(22) Date of filing: 22.09.2009
(51) Int. Cl.: A61P 33/06, A61K 31/55, C07D 223/14, C07D 401/04, C07D 403/04

(54) **Novel antimalarial 3-azabicyclo[3.2.2]nonane derivatives**

(71) Applicant: University of Graz, 8010 Graz (AT)
(72) Inventor: Seebacher, Werner, 8044 Graz-Mariatrost (AT); Weis, Robert, 8046 Stattegg (AT)
(74) Representative: Patentanwaltskanzlei Matschnig & Forsthuber OG

(57) **Abstract**

The present invention relates to novel 3-azabicyclo[3.2.2]nonane derivatives of the general formula (I) 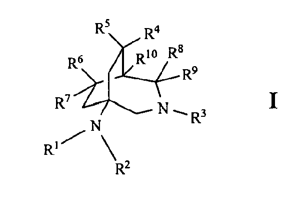 or a pharmaceutically acceptable salt thereof
wherein R¹, R², R³, R⁴, R⁵, R⁶, R⁷, R⁸, R⁹ and R¹⁰ are as defined in the specification. The novel 3-azabicyclo[3.2.2]nonane derivatives are particularly useful for treatment and prevention of malaria and trypanosomiasis.

## Description

### FIELD OF THE INVENTION

The present invention relates to novel 3-azabicyclo[3.2.2]nonane derivatives of the general formula **I** 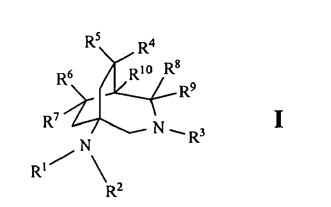 or a pharmaceutically acceptable salt thereof
wherein
R¹ and R² each independently are
   (1) hydrogen
   (2) C₁-C₁₀alkyl, optionally substituted with a substituent containing a nitrogen atom or with an optionally substituted 3 to 7 membered non-aromatic heterocyclic ring,
   (3) an acyl group selected from
      an optionally substituted C₁-C₅alkylcarbonyl,
      an optionally substituted C₅-C₁₄arylcarbonyl,
      an optionally substituted C₅-C₁₄aryl C₁-C₅alkylcarbonyl,
      an optionally substituted 6 to 10 membered heteroarylcarbonyl, and
      an optionally substituted 6 to 10 membered heteroaryl-C₁-C₅alkylcarbonyl,
   (4) C₆-C₁₄aryl-C₁-C₄alkyl, or
   (5) heteroaryl-C₁-C₄alkyl;
   or
R¹ and R², taken together with the nitrogen, form a 3 to 7 membered non-aromatic heterocyclic ring which may be substituted or may contain one or more additional heteroatoms;
R³ is selected as defined above for R1 and R2 according to (1) to (5);
R⁴ is hydrogen, C₁-C₁₀alkyl, optionally substituted C₆-C₁₀aryl or optionally substituted 5 to 10 membered heteroaryl;
R⁵ is hydrogen;
R⁶ is hydrogen, C₁-C₁₀alkyl, optionally substituted C₆-C₁₀aryl or optionally substituted 5 to 10 membered heteroaryl; R⁶ and R⁴ can be identical or can differ from each other;
R⁷ is hydrogen, C₁-C₁₀alkyl, optionally substituted C₆-C₁₀aryl or optionally substituted 5 to 10 membered heteroaryl;
R⁸ and R⁹ are either hydrogen or together a double bonded oxygen; and
R¹⁰ is hydrogen, C₁-C₁₀alkyl being optionally substituted with a substituent containing a nitrogen atom or with an optionally substituted 3 to 7 membered non-aromatic heterocyclic ring, optionally substituted C₆-C₁₄aryl or optionally substituted heteroaryl.

The novel 3-azabicyclo[3.2.2]nonane derivatives are particularly useful for treatment and prevention of malaria and trypanosomiasis.

### BACKGROUND OF THE INVENTION

Malaria is an infectious disease caused by the protozoan parasites of the genus *Plasmodium.* Two to three million people die from it annually, and children are particularly affected. The most deadly and virulent strain causing malaria tropica is *Plasmodium falciparum.* Its widespread drug resistance is the main reason for increasing malaria deaths. Therefore, chloroquine and the combination sulfadoxine/pyrimethamine, which were once active against *Plasmodium falciparum,* are today almost useless in many parts of the world. Increasing resistance has also been reported for the subsequent generation of antimalarials, and loss of sensitivity of the most recently introduced artemisine derivatives has been observed both *in vitro* and *in vivo.* Hence, there is great demand for the development of new antiplasmodial drugs.

Human African trypanosomiasis is caused by protozoans, and about 0.5 million people in central Africa are infected with *Trypanosoma brucei gambiense* or *Trypanosoma brucei rhodesiense.* This disease causes 50.000 deaths per year and is fatal if untreated. Only four drugs, pentamidine, suramine, melarsoprol, and eflornithine, are available for treatment of this disease, which starts with a peripheral stage and progresses to a CNS infection, the late stage of sleeping sickness. Pentamidine and suramine are only suitable for treatment of the peripheral stage because they are not able to cross the blood-brain barrier. Melarsopol, which is active in all stages against all strains of trypanosomes, can cause severe, usually fatal encephalopathy for up to 5% of patients (WHO, 1999). Therefore, it is usually substituted by eflornithine against the late stage of West African Trypanosomiasis, which is caused by *Trypanosoma brucei gambiense.* However, it is not active against *Trypanosoma brucei rhodesiense,* the causative organism of East African Trypanosomiasis. Therefore, only a single compound, melarsopol, which can cause severe side effects, is available for treatment of late-stage East African Trypanosomiasis, which is why new trypanocides, particularly against *Trypanosoma brucei rhodesiense,* are needed.

Recently derivatives based on 2-azabicyclo[3.2.2]nonane were described ("Seebacher et al., 2005, Synthesis of 2-azabicyclo[3.2.2]nonanes from bicyclo[2.2.2.]octan-2-ones and their activities against Trypanosoma brucei rhodesiense and Plasmodium falciparum K1, Journal of Pharmacy and Pharmaceutical Sciences 8:578-585"; "Weis et al., 2008, Synthesis of bicyclic amines and their activities against Trypanosoma brucei rhodesiense and Plasmodium falciparum K1, Archives of Pharmacal Research 31:688-697"; "Berger et al., 2008, Novel Azabicyclo[3.2.2.]nonane derivatives and their activities against Plasmodium falciparum K1 and Trypanosoma b. rhodesiense, Bioorganic and Medicinal Chemistry 16:6371-6378"; "Seebacher et al., 2007, New 4-Amino-2-azabicyclo[3.2.2]nonane derivatives and their antiprotozoal potencies, Monatshefte für Chemie 138:619-625", "Faist et al., 2009, Antiplasmodial and antitrypanosomal activity of bicyclic amides and esters of dialkylamino acids, Bioorganic & Medicinal Chemistry 17: 3595-3603", "Weis et al., 2008, Acyl derivatives of 5-amino-2-azabicyclo[3.2.2]nonanes, Monatshefte für Chemie 139: 717-724", "Seebacher et al., 2007, Antiprotozoal Activities of Epimeric Aminobicycles, Monatshefte für Chemie 138: 709-714", "Berger et al., 2006, Antiprotozoal activities of new bis-chlorophenyl derivatives, Bioorganic & Medicinal Chemistry Letters 16: 5457-5461", "Weis et al., 2009, New Bicyclic Amines: Synthesis and SARs of their Action Against the Causative Organisms of Malaria and Sleeping Sickness, Current Medicinal Chemistry 16: 1426-1441"). However, these compounds were only effective *in vitro* against the causative organisms of malaria and the causative organism of sleeping sickness but lacked *in vivo* activity..

### DESCRIPTION OF THE INVENTION

A main object of the present invention is to provide novel 3-azabicyclo[3.2.2]nonane derivatives of the general formula I as defined above and pharmaceutical acceptable salts thereof for therapeutic use. The novel 3-azabicyclo[3.2.2]nonane derivatives are particularly useful for treatment, suppression and prevention of diseases related to both chloroquine-resistant and chloroquine-sensitive strains of the causative organisms of malaria as well as the causative organism of sleeping sickness.

It is a further object of the present invention to provide a pharmaceutical composition comprising at least one 3-azabicyclo[3.2.2]nonane derivative of general formula I as an active ingredient.

The object of the present invention is also to provide methods for the preparation of the 3-azabicyclo[3.2.2]nonane derivatives of the general formula I. The 3-azabicyclo[3.2.2]nonane derivatives of the present invention have the following general formula (formula I): 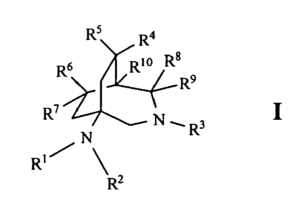
wherein the residues R¹, R², R³, R⁴, R⁵, R⁶, R⁷, R^{8,} R⁹ and R¹⁰ are as defined above.

The term "alkyl" as used herein refers to straight or branched hydrocarbon chains having a specified number of carbon atoms. For example, C₁-C₁₀alkyl refers to a straight or branched alkyl group having at least 1 and at most 10 carbon atoms. Alkyl groups having a straight chain preferably represent C₁-C₇alkyl. Useful branched chains, which preferably represent C₃-C₁₀alkyl, are for example isopropyl, isobutyl, sec. butyl, tert. butyl, pentan-1-yl, pentan-2-yl, pentan-3-yl and 6-ethyloctan-2-yl groups.

The term "alkyl being substituted with a substituent containing a nitrogen atom" as used herein for R¹, R², R³ and R¹⁰, respectively, refers to an "alkyl" as defined above bearing an additional nitrogen-containing substituent. The substituent containing a nitrogen atom is preferably selected from primary, secondary and tertiary amines, and cyano. Useful examples of alkyl groups which are substituted with a substituent containing a nitrogen atom include for example diethylaminoethyl, diethylaminopropyl, diethylaminobutyl groups as well as dipropylaminoethyl, dipropylaminopropyl, dipropylaminobutyl, diisopropylaminoethyl, diisopropylaminopropyl, diisopropylaminobutyl, dibutylaminoethyl, dibutylaminopropyl and dibutylaminobutyl moieties. Preferred are 5-diethylaminopentan-2-yl residues.

The term "alkyl being substituted with a non-aromatic heterocyclic ring" as used herein for R¹, R², R³ and R¹⁰, respectively, refers to an alkyl as defined above being substituted with a monocyclic preferably 3-7-membered saturated hydrocarbon ring containing at least one heteroatom independently selected from oxygen and nitrogen. The heterocyclic ring may be substituted, wherein preferred substituents are selected from methyl or hydroxy. Useful examples of alkyl residues being substituted with a non-aromatic heterocyclic ring include, but are not limited to pyrrolidinoethyl, pyrrolidinopropyl, pyrrolidinobutyl, piperdinoethyl, piperidinopropyl, piperidinobutyl, 4-methylpiperazinoethyl, 4-methylpiperazinopropyl, 4-methylpiperazinobutyl, 4-hydroxypiperidinoethyl, 4-hydroxypiperidinopropyl, 4-hydroxypiperidinobutyl, azepan-1-ylethyl, azepan-1-ylpropyl, azepan-1-ylutyl, aziridin-1-ylethyl, aziridin-1-ylpropyl, aziridin-1-ylbutyl, azetidin-1-ylethyl, azetidin-1-ylpropyl and azetidin-1-ylbutyl residues.

The term "aryl" as used herein refers to an aromatic carbocyclic ring having a specified number of carbon atoms. For example, C₅-C₆aryl refers to an aromatic ring having at least 5 and at most 6 carbon atoms. The term "aryl" also refers to a multicyclic group having more than one aromatic ring, such as C₁₀-C₁₄aryl. Useful examples are cyclopentadienyl, phenyl, naphthyl, anthracyl, phenantryl.

The term "heteroaryl" as used herein refers to aromatic groups having one or more heteroatoms (O, S or N). A multicyclic group having one or more heteroatoms, wherein at least one ring of the group is heteroaromatic, is referred to as a "heteroaryl" as well.

The term "acyl" as used herein for R¹, R² and R³, respectively, refers to compounds of the general formula R-(C=O)- wherein R may be alkyl ("alkylcarbonyl"), aryl ("arylcarbonyl"), arylalkyl ("arylalkylcarbonyl"), heteroaryl ("heteroarylcarbonyl"), or heteroarylalkyl ("heteroarylalkylcarbonyl") wherein the following acyl groups are useful:
Useful "alkylcarbonyl" groups, preferably C₁-C₅alkylcarbonyl, include for example acetyl propionyl butanoyl pentanoyl. The alkyl carbonyl may be substituted with a halogen, wherein particularly chloro-alkylcarbonyl compounds like 2-chloroacetyl, 3-chloropropionyl, 4-chlorobutanoyl and 5-chloropentanoyl are useful examples.

Useful "arylcarbonyl" residues are for example benzoyl, naphthalene-1-carbonyl, naphthalene-2-carbonyl, cyclopentadiene-carbonyl, cycloheptadiene-carbonyl, anthracene-carbonyl, and phenanthrene-carbonyl groups. The aryl group is preferably C₅-C₁₄aryl. Useful "arylalkylcarbonyl" residues preferably represent C₅-C₁₄aryl-C₁-C₅alkylcarbonyl such as phenylethanoyl, 3-phenylpropanoyl, 4-phenylbutanoyl, naphth-1-ylethanoyl, 3-(1-naphth-1-yl)propanoyl. The aryl group of the arylcarbonyl residue and the arylalkylcarbonyl residue, respectively may be substituted. Typical substituted aryl groups are 4-chlorophenyl, 4-bromophenyl, 4-fluorophenyl, 4-trifluoromethylphenyl, 4-methylphenyl, 4-methoxyphenyl, 4-isopropylphenyl, 6-fluoro-naphthalen-1-yl, 6-chloro-naphtalen-1-yl, 6-methoxy-naphthalen-1-yl, 6-trifluoromethyl-naphthalen-1-yl, 3-methoxy-1-naphthalen-1-yl, 3-chloro-naphthalen-1-yl, 3-trifluoromethyl-naphthalen-1-yl, 3-fluoro-naphthalen-1-yl, 3,5-dichlorophenyl, 3,4-dichlorophenyl, 2-chlorophenyl, 2,6-dichlorophenyl, 3,5-difluoro-naphthalen-1-yl, 3,5-dichloro-naphthalen-1-yl, 3,5-dimethoxy-naphthalen-1-yl, 3,5-bis(trifluoromethyl)naphthalen-1-yl, 4-nitrophenyl, and 4-aminophenyl.

Useful "heteroarylcarbonyl" residues, wherein the heteroaryl group preferably has 6 to 10 members, are for example nicotinyl, isonicotinyl, picolinyl, pyrrol-2-carbonyl, pyrrol-3-carbonyl, quinoline-2-carbonyl, quinoline-3-carbonyl, quinoline-4-carbonyl, isoquinoline-1-carbonyl, isoquinoline-3-carbonyl, isoquinoline-4-carbonyl, ferrocene-carbonyl. Moreover, the acyl group may be a heteroarylalkylcarbonyl group, wherein a C₁-C₅alkyl chain is located between the heteroaryl and the carbonyl carbon; a useful example is indol-3-yl acetyl. The heteroaryl group of the heteroarylcarbonyl residue and the heteroarylalkyl residue, respectively, may be substituted. Typical substituted heteroaryl residues are 6-chloro-quinolin-1-yl, 6-fluoro-quinolin-1-yl, 6-methoxy-quinolin-1-yl, 6-trifluoromethyl-quinolin-1-yl, 7-chloro-quinolin-1-yl, 7-fluoro-quinolin-1-yl, 7-methoxy-quinolin-1-yl, 7-trifluoromethyl-quinolin-1-yl, 8,chloro-quinolin-1-yl, 8-fluoro-quinolin-1-yl, 8-methoxy-quinolin-1-yl, 8-trifluoromethyl-quinolin-1-yl substituents.

Useful "aryl alkyl" groups, wherein the aryl preferably is a C₆-C₁₄aryl and the alkyl is preferably a C₁-C₄alkyl, include for example benzyl, naphthalen-1-yl-methyl, naphthalen-2-yl-methyl, cyclopentadienyl-methyl, cycloheptadienyl-methyl, anthracene-methyl, and phenanthrene-methyl groups. Furthermore, phenylethyl, phenylpropyl and phenylbutyl groups are considered.

Useful "heteroaryl alkyl" groups, wherein the heteroaryl preferably is a selected from a 5 to 10 membered heterocyclic ring and alkyl preferably represents C₁-C₄alkyl include for example pyridin-4-ylmethyl, pyridin-3-ylmethyl, pyridin-2-ylmethyl, pyridin-4-ylpropyl, pyridin-4-ylbutyl, pyrrol-2-ylmethyl, pyrrol-3-ylmethyl, indole-3-ylmethyl, indol-3-ylethyl and ferrocene-methyl substituents.

If R⁴, R⁶ and/or R⁷ represent aryl or heteroaryl, C₆-C₁₀aryl and 5 to 10 membered heteroaryl, respectively, are preferred embodiments. The aryl or heteroaryl group may be substituted. Preferred substituents are selected from the group consisting of halogen, nitro, amino, alkyl or alkoxy.

R⁶ and R⁴ can be identical or can differ from each other. Moreover, R⁷ and R⁵ can be identical or can differ from each other.

In a particularly preferred embodiment, R⁴ and R⁶ each independently represent phenyl being optionally substituted with 1 to 5 substituents selected from the group consisting of halogen, methyl and amino, R⁷ represents H and all other residues are as defined above in general formula I. Halogen means chlorine, bromine, fluorine or iodine.

Typical substituted aryl residues, which R⁴, R⁶ and R⁷, respectively, may represent, are 4-chlorophenyl, 4-bromophenyl, 4-fluorophenyl, 4-trifluoromethylphenyl, 4-methylphenyl, 4-methoxyphenyl, 4-isopropylphenyl, 6-fluoro-naphthalen-1-yl, 6-chloro-naphtalen-1-yl, 6-methoxy-naphthalen-1-yl, 6-trifluoromethyl-naphthalen-1-yl, 3-methoxy-1-naphthalen-1-yl, 3-chloro-naphthalen-1-yl, 3-trifluoromethyl-naphthalen-1-yl, 3-fluoro-naphthalen-1-yl, 3,5-dichlorophenyl, 3,4-dichlorophenyl, 2-chlorophenyl, 2,6-dichlorophenyl, 3,5-difluoro-naphthalen-1-yl, 3,5-dichloro-naphthalen-1-yl, 3,5-dimethoxy-naphthalen-1-yl, 3,5-bis(trifluoromethyl)naphthalen-1-yl, 4-nitrophenyl, and 4-aminophenyl.

Typical substituted heteroaryl residues, which R⁴, R⁶ and R⁷, respectively, may represent, are 6-chloro-quinolin-1-yl, 6-fluoro-quinolin-1-yl, 6-methoxy-quinolin-1-yl, 6-trifluoromethyl-quinolin-1-yl, 7-chloro-quinolin-1-yl, 7-fluoro-quinolin-1-yl, 7-methoxy-quinolin-1-yl, 7-trifluoromethyl-quinolin-1-yl, 8,chloro-quinolin-1-yl, 8-fluoro-quinolin-1-yl, 8-methoxy-quinolin-1-yl, 8-trifluoromethyl-quinolin-1-yl substituents.

In one embodiment R¹ and R² may together with the nitrogen form a non-aromatic heterocyclic ring. Preferably the heterocyclic ring comprises 3 to 7 members. Moreover, the heterocyclic ring may be substituted and/ or may contain in addition to the nitrogen one or more heteroatoms selected from the group consisting of nitrogen and oxygen and N-R¹¹, wherein R¹¹ is preferably selected from hydrogen and C₁-C₄alkyl, preferably methyl. In one particularly preferred embodiment R¹ and R² taken together with the nitrogen preferably form a 3 to 7 membered heterocyclic ring selected from pyrrolidine, piperidine, morpholine, N-methylpiperazine, azepane, aziridine and azetidine, more preferably a 5 to 6 membered heterocyclic ring selected from pyrrolidine, piperidine, morpholine and N-methylpiperazine. The heterocyclic ring may be substituted with one ore more substituents, wherein the substituent is preferably selected from C₁-C₁₀alkyl and amino.

R¹⁰ is hydrogen but can also be C₁-C₁₀alkyl, optionally substituted with a substituent containing a nitrogen atom or with an optionally substituted 3 to 7 membered non-aromatic heterocyclic ring, a C₆-C₁₄aryl or heteroaryl residue. If R¹⁰ is a heteroaryl residue, it is preferably a 5 to 10 membered heteroaryl residue. Both the aryl and the heteroaryl residue may be substituted as described above for R⁴, R⁶ and R⁷.

In another embodiment of the invention, at least one of the residues R¹, R², R³, R⁴, R⁶, R⁷, R⁸, R⁹ and R¹⁰ does not represent hydrogen and the other residues represent hydrogen. Preferably at least one of R¹, R², R⁴, R⁶ and R⁷ does not represent hydrogen and the other residues represent hydrogen. Most preferably at least one of R¹, R⁴ and R⁶ does not represent hydrogen and the other residues represent hydrogen.

In one preferred embodiment, only R⁴ does not represent hydrogen and the other residues represent hydrogen. In another preferred embodiment, R¹, R², R⁴ and R⁶ do not represent hydrogen and the other residues represent hydrogen.

The term "substituted" refers to a chemical entity being substituted with one or more substituents. The term "optionally substituted" relating to a chemical entity means that the chemical entity may be substituted or not, i.e. both situations are included. The term "one or more substituents" refers to from one to the maxium number of substituents possible, depending on the number of available substitution sites.

Certain 3-azabicyclo[3.2.2]nonanes of general formula I may exist in different stereoisomeric forms, wherein the individual stereoisomers, including enantiomers and diastereoisomers, and mixtures thereof are also within the scope of the present invention. Individual isomers can be obtained by methods known to those skilled in the art. Individual isomers and mixtures thereof as well as racemic mixtures are included as well.

Diamine-based compounds were found to be very effective, in particular with regard to treatment of malaria and sleeping sickness. Therefore, in a particularly preferred embodiment of the invention, the compound of general formula I is selected from
(6RS,9RS)-(±)-1-Dimethylamino-6,9-diphenyl-3-azabicyclo[3.2.2]nonane
(6RS,9RS)-(±)-1-Morpholino-6,9-diphenyl-3-azabicyclo[3.2.2]nonane
(6RS,9RS)-(±)-6,9-Diphenyl-1-pyrrolidino-3-azabicyclo[3.2.2]nonane
(6RS,9RS)-(±)-6,9-Diphenyl-1-piperidino-3-azabicyclo[3.2.2]nonane
(6RS,9RS)-(±)-1-(4-Methylpiperazin-1-yl)-6,9-diphenyl-3-azabicyclo[3.2.2]nonane
(6RS,9RS)-(±)-6,9-Bis(4-chlorophenyl)-1-dimethylamino-3-azabicyclo[3.2.2]nonane
(6RS,9RS)-(±)-6,9-Bis(4-chlorophenyl)-1-pyrrolidino-3-azabicyclo[3.2.2]nonane
(6RS,9RS)-(±)-6,9-Bis(4-chlorophenyl)-1-piperidino-3-azabicyclo[3.2.2]nonane
(6RS,9RS)-(±)-1-Dimethylamino-6,9-bis(4-methylphenyl)-3-azabicyclo[3.2.2]nonane
(6RS,9RS)-(±)-6,9-Bis(4-methylphenyl)-1-pyrrolidino-3-azabicyclo[3.2.2]nonane
(6RS,9RS)-(±)-6,9-Bis(4-methylphenyl)-1-piperidino-3-azabicyclo[3.2.2]nonane
(6RS,9RS)-(±)-1-Methylamino-6,9-diphenyl-3-azabicyclo[3.2.2]nonane
(6RS,9RS)-(±)-6,9-Bis(2-chlorophenyl)-1-dimethylamino-3-azabicyclo[3.2.2]nonane
(6RS,9RS)-(±)-6,9-Bis(2-chlorophenyl)-1-pyrrolidino-3-azabicyclo[3.2.2]nonane
(6RS,9RS)-(±)-6,9-Bis(2-chlorophenyl)-1-piperidino-3-azabicyclo[3.2.2]nonane
(6RS,9RS)-(±)-1-(N-(2-Diethylaminoethyl)-N-methylamino)-6,9-diphenyl-3-azabicyclo[3.2.2]nonane
(6RS,9RS)-(±)-1-(N-(2-Dipropylaminoethyl)-N-methylamino)-6,9-diphenyl-3-azabicyclo[3.2.2]nonane
(6RS,9RS)-(±)-1-(N-Methyl-N-(2-pyrrolidinoethyl)amino)-6,9-diphenyl-3-azabicyclo[3.2.2]nonane
(6RS,9RS)-(±)-1-(N-Methyl-N-(2-piperidinoethyl)amino)-6,9-diphenyl-3-azabicyclo[3.2.2]nonane
(6RS,9RS)-(±)-1-(N-Methyl-N-(2-(4-methylpiperazin-1-yl)ethyl)amino)-6,9-diphenyl-3-azabicyclo[3.2.2]nonane
(6RS,9RS)-(±)-3-Ethylamino-6,9-diphenyl-1-pyrrolidino-3-azabicyclo[3.2.2]nonane
(6RS,9RS)-(±)-1-(N-Methyl-N-(pyridin-3-ylmethyl)amino)-6,9-diphenyl-3-azabicyclo[3.2.2]nonane
(6RS,9RS)-(±)-6-(4-Aminophenyl)-9-phenyl-1-pyrrolidino-3-azabicyclo[3.2.2]nonane
(6RS,9RS)-(±)-1-(N-(3-Aminopropyl)-N-methylamino)-6,9-diphenyl-3-azabicyclo[3.2.2]nonane
5-Phenyl-1-pyrrohdino-3-azabicyclo[3.2.2]nonane

In another preferred embodiment of the invention, the compound of general formula I may be amide-based and is preferably selected from
(7RS,8RS)-(±)-5-Dimethylamino-7,8-diphenyl-3-azabicyclo[3.2.2]nonan-2-one
(7RS,8RS)-(±)-5-Morpholino-7,8-diphenyl-3-azabicyclo[3.2.2]nonan-2-one
(7RS,8RS)-(±)-7,8-Diphenyl-5-pyrrolidino-3-azabicyclo[3.2.2]nonan-2-one
(7RS,8RS)-(±)-7,8-Diphenyl-5-piperidino-3-azabicyclo[3.2.2]nonan-2-one
(7RS,8RS)-(±)-5-(4-Methylpiperazin-1-yl)-7,8-diphenyl-3-azabicyclo[3.2.2]nonan-2-one
(7RS,8RS)-(±)-7,8-Bis(4-chlorophenyl)-5-dimethylamino-3-azabicyclo[3.2.2]nonan-2-one
(7RS,8RS)-(±)-7,8-Bis(4-chlorophenyl)-5-pyrrolidino-3-azabicyclo[3.2.2]nonan-2-one
(7RS,8RS)-(±)-7,8-Bis(4-chlorophenyl)-5-piperidino-3-azabicyclo[3.2.2]nonan-2-one
(7RS,8RS)-(±)-5-Dimethylamino-7,8-bis(4-methylphenyl)-3-azabicyclo[3.2.2]nonan-2-one
(7RS,8RS)-(±)-7,8-Bis(4-methylphenyl)-5-pyrrolidino-3-azabicyclo[3.2.2]nonan-2-one
(7RS,8RS)-(±)-7,8-Bis(4-methylphenyl)-5-piperidino-3-azabicyclo[3.2.2]nonan-2-one
(7RS,8RS)-(±)-5-Methylamino-7,8-diphenyl-3-azabicyclo[3.2.2]nonan-2-one
(7RS,8RS)-(±)-7,8-Bis(2-chlorophenyl)-5-dimethylamino-3-azabicyclo[3.2.2]nonan-2-one
(7RS,8RS)-(±)-7,8-Bis(2-chlorophenyl)-5-pyrrolidino-3-azabicyclo[3.2.2]nonan-2-one
(7RS,8RS)-(±)-7,8-Bis(2-chlorophenyl)-5-piperidino-3-azabicyclo[3.2.2]nonan-2-one
(6RS,9RS)-(±)-2-Diethylamino-N-methyl-N-(4-oxo-6,9-diphenyl-3-azabicyclo[3.2.2]non-1-yl)acetamide
(6RS,9RS)-(±)-2-Di-(n-propylamino)-N-methyl-N-(4-oxo-6,9-diphenyl-3-azabicyclo[3.2.2]non-1-yl)acetamide
(6RS,9RS)-(±)-N-Methyl-N-(4-oxo-6,9-diphenyl-3-azabicyclo[3.2.2]non-1-yl)-2-pyrrolidino-acetamide
(6RS,9RS)-(±)-N-Methyl-N-(4-oxo-6,9-diphenyl-3-azabicyclo[3.2.2]non-1-yl)-2-piperidino-acetamide
(6RS,9RS)-(±)-N-Methyl-2-(4-methylpiperazin-1-yl)-N-(4-oxo-6,9-diphenyl-3-azabicyclo[3.2.2]non-1-yl)acetamide
(6RS,9RS)-(±)-2-Cyano-N-methyl-N-(4-oxo-6,9-diphenyl-3-azabicyclo[3.2.2]non-1-yl)acetamide
(6RS,9RS)-(±)-3-(2-Aminoethyl)-6,9-diphenyl-1-pyrrolidino-3-azabicyclo[3.2.2]nonane
(6RS,9RS)-(±)-3-(2-Diethylaminoethyl)-1-dimethylamino-6,9-diphenyl-3-azabicyclo[3.2.2]nonane
(6RS,9RS)-(±)-3-(2-Diethylaminoethyl)-6,9-diphenyl-1-pyrrolidino-3-azabicyclo[3.2.2]nonane
(6RS,9RS)-(±)-3-(2-Diethylaminoethyl)-6,9-diphenyl-1-piperidino-3-azabicyclo[3.2.2]nonane 1-Phenyl-5-pyrrolidino-3-azabicyclo[3.2.2]nonan-2-one

In one aspect of the invention compounds of general formula **I** are provided which are useful as therapeutically active substances, e.g. in form of a medicament.

The compounds of general formula **I** can be particularly useful as therapeutically active substances for treating and preventing diseases and conditions associated with **plasmodial** strains, in particular malaria and trypanosomiasis.

A further feature of the invention is a pharmaceutical composition comprising at least one compound of general formula **I** or a pharmaceutically acceptable salt thereof as an active ingredient.

Moroever, the invention relates to a pharmaceutical composition comprising as an active ingredient at least one compound of the general formula **I** or a pharmaceutically acceptable salt thereof and at least one of a pharmaceutically acceptable carrier and/or excipient.

Several compounds according to the invention were tested for their activity against causative organisms of malaria and sleeping sickness. The tested compounds showed a distinct *in vitro* activity comparable to the activity observed for chloroquine against *Plasmodium falciparum K₁* as well as a distinct *in vivo* activity against *Plasmodium berghei* in a mouse model. Moreover, the compounds exhibited a low cytotoxicity. Some compounds showed distinct *in vitro* activity against causative organisms of sleeping sickness, therefore the tested compounds are valuable leads for the development of new agents for treatment of sleeping sickness.

Accordingly, a further feature of the invention is a pharmaceutical composition for treating or preventing a disorder or condition selected from malaria and sleeping sickness, comprising an amount of a compound according to the invention, or a pharmaceutically acceptable salt thereof that is effective in treating such disorder or condition and a pharmaceutically acceptable carrier and/or excipient.

The compounds of the invention can be used for the development of a medicament, wherein the medicament is particularly useful for treating or preventing a disease or condition associated with strains selected from chloroquine-resistant or chloroquine-sensitive strains of the causative organisms of malaria and the causative organism of sleeping sickness. The causative organisms of sleeping sickness include *Trypanosoma brucei brucei* and *Trypanosoma brucei rhodesiense.* The causative organisms of malaria include chloroquine-resistant and chloroquine-sensitive strains of *Plasmodium falciparum.*

Another aspect of the invention relates to a method of treating a subject suffering from malaria and trypanosomiasis, respectively, comprising administering to the subject a pharmaceutically effective amount of at least one of the 3-azabicyclo[3.2.2]nonane derivatives according to the invention. In one embodiment of the invention, the subject is a human being.

Depending on the specific condition or disease state to be treated, the 3-azabicyclo[3.2.2]nonane derivatives according to the present invention may be administered to subjects at any appropriate therapeutically effective and safe dosage. The dosage may be determined by means of methods known by those skilled in the art.

Advantageously, the compounds and the pharmaceutical composition, respectively, may be intended for oral administration, e.g. in form of tablets, pills, capsules or troches, but also in liquid form such as tonics, syrups, suspensions or drops. Moreover, it is also possible to administer the compounds or the pharmaceutical composition parenterally (e.g. intravenously, intramuscularly, intraperitoneally, subcutaneously, intracutaneously) or topically.

In another aspect, the invention relates to methods for producing 3-azabicyclo[3.2.2]nonane derivatives of the general formula I, which methods are described in the following:
*Method 1:* If R¹ and R² each independently represent C₁-C₁₀alkyl being optionally substituted with a substituent containing a nitrogen atom or with an optionally substituted 3-7 membered non-aromatic heterocyclic ring, C₆-C₁₄aryl-C₁-C₄alkyl, or heteroaryl-C₁-C₄alkyl; or R¹ and R², taken together with the nitrogen, form a 3 to 7 membered non-aromatic heterocyclic ring which may be substituted or may contain one or more additional heteroatoms; R³ =H; R⁴ = R⁶ = aryl or heteroaryl; R⁵ = R⁷ = R¹⁰ = H, the 3-azabicyclo[3.2.2]nonane derivatives with the general formula **I** can be synthesized from benzylidene acetone or substituted benzylidene acetones or other 4-aryl or 4-heteroaryl substituted but-3-en-2-ones, which are commercial available or accessible by known or reported procedures and hydrothiocyanates of secondary amines, which are commercial available or accessible by known or reported procedures, in a boiling solvent like toluene, dimethylformamide or isopropanol with water separation or without use of a solvent giving **III**. The next step is a Schmidt-reaction to **II** where **III** is dissolved in cold concentrated sulfuric acid and treated with sodium azide. The reduction of **II** to **I** can be done with lithium aluminium hydride in dry diethyl ether or other solvents like tetrahydrofuran or dioxane or with other hydrides or other hydrogenation catalysts. 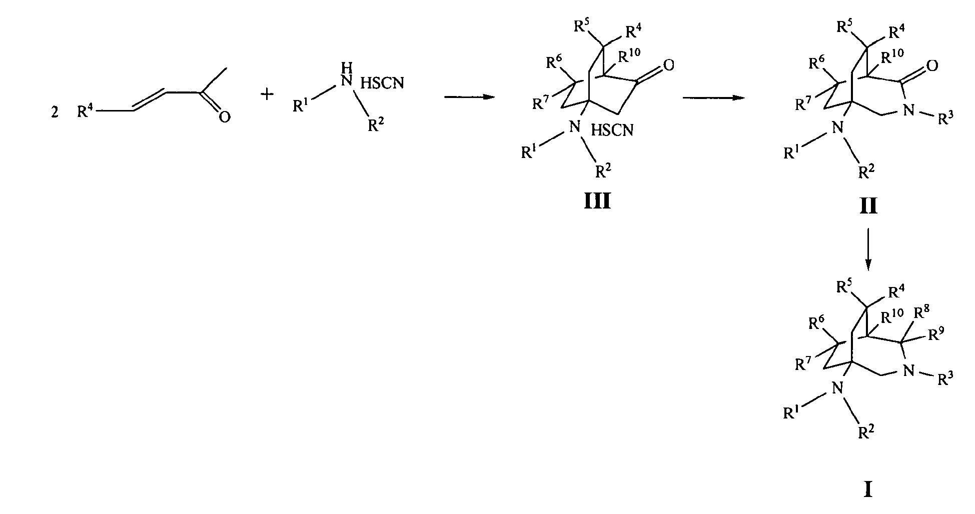
*Method 2:* If R¹ and R² each independently represent C₁-C₁₀alkyl being optionally substituted with a substituent containing a nitrogen atom or with an optionally substituted 3-7 membered non-aromatic heterocyclic ring, C₆-C₁₄aryl-C₁-C₄alkyl, or heteroaryl-C₁-C₄alkyl; or R¹ and R², taken together with the nitrogen, form a 3 to 7 membered non-aromatic heterocyclic ring which may be substituted or may contain one or more additional heteroatoms; R³ =H; R⁴ = R⁷ = aryl or heteroaryl; R⁵ = R⁶ = R¹⁰ = H, compounds **IV** can be prepared as mentioned above for compounds **III.** Then an izomerization of **IV** to **V** succeeds in the presence of alkaline catalysts such as potassium tert. butylate under heating with or without solvent can be done. Schmidt reaction from **V** to **VI** can be done as described above as well as the subsequent hydrogenation to the 3-azabicyclo[3.2.2.]nonane derivatives with the general formula **I.** 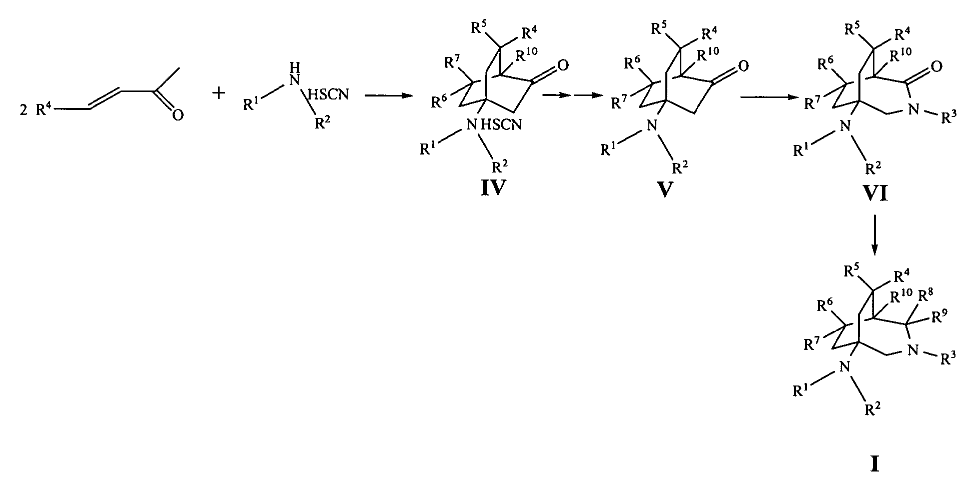
*Method* 3: If R¹ and R² each independently represent C₁-C₁₀alkyl being optionally substituted with a substituent containing a nitrogen atom or with an optionally substituted 3-7 membered non-aromatic heterocyclic ring, C₆-C₁₄aryl-C₁-C₄alkyl, or heteroaryl-C₁-C₄alkyl; or R¹ and R², taken together with the nitrogen, form a 3 to 7 membered non-aromatic heterocyclic ring which may be substituted or may contain one or more additional heteroatoms; R³ =H; R⁴ ≠ R⁶ = H, alkyl, aryl or heteroaryl as defined above; R⁵ = R⁷ = H, R¹⁰ = H, alkyl, aryl or heteroaryl as defined above, the 3-azabicyclo[3.2.2]nonane derivatives with the general formula **I** can be synthesized from silylated (R = alkyl) benzylidene acetone or substituted benzylidene acetones or 2-(trialkylsilyloxy)-1,3-butadienes or other silylated 4-alkyl, 4-aryl or 4-heteroaryl substituted but-3-ene-2-ones, which are commercial available or accessible by known or reported procedures and benzylidene acetones and (substituted) benzylidene acetones or 1,3-butadiene or other 4-alkyl, 4-aryl or 4-heteroaryl substituted but-3-ene-2-ones, which are commercial available or accessible by known or reported procedures giving unsymmetric substituted diketones **VII** via a Diels-Alder procedure. Alternatively, if R¹ and R² each independently represent C₁-C₁₀alkyl being optionally substituted with a substituent containing a nitrogen atom or with an optionally substituted 3-7 membered non-aromatic heterocyclic ring, C₆-C₁₄aryl-C₁-C₄alkyl, or heteroaryl-C₁-C₄alkyl; or R¹ and R², taken together with the nitrogen, form a 3 to 7 membered non-aromatic heterocyclic ring which may be substituted or may contain one or more additional heteroatoms; R³ =H; R⁴ = R⁶ = H, alkyl, aryl or heteroaryl as defined above; R⁵ = R⁷ = H, R¹⁰ = alkyl, aryl or heteroaryl as defined above, **VIIa** can be produced by the cyclization of a pimelic acid derivative. Compounds of formula **VII** and **VIIa** can be identical. **VII** and **VIIa,** respectively react with secondary amines to **VIII.** Its hydrochloride can be converted to **IX** by a Schmidt reaction as described above. The hydrogenation of lactams **IX** yields compounds **I** as described above. 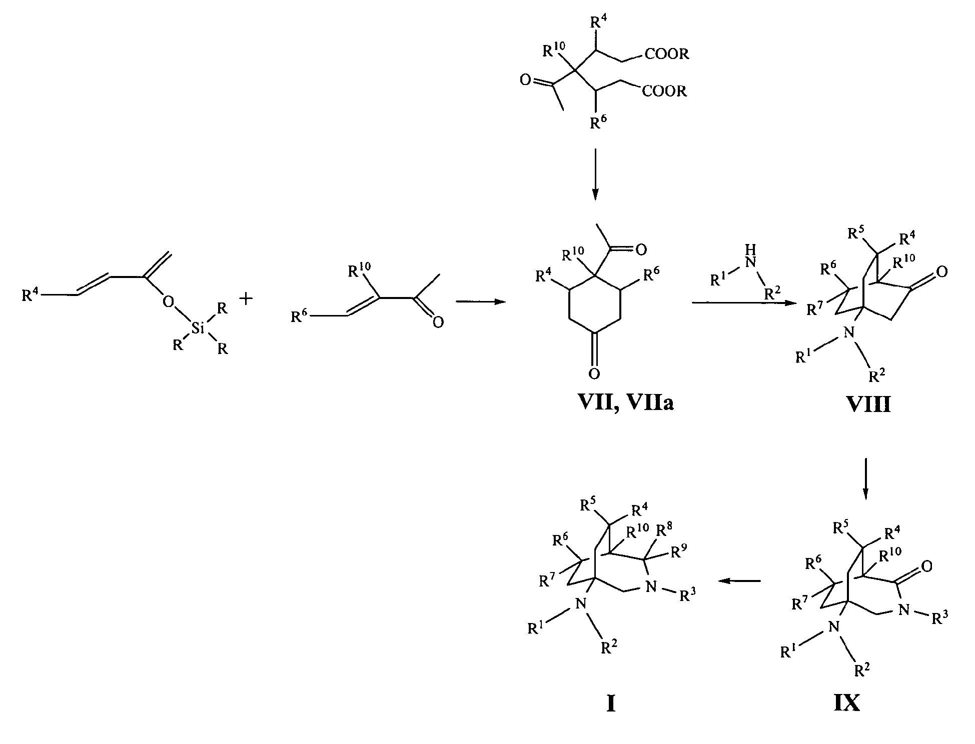
*Method 4:* If R¹ and R² each independently represent hydrogen, C₁-C₁₀alkyl being optionally substituted with a substituent containing a nitrogen atom or with an optionally substituted 3-7 membered non-aromatic heterocyclic ring, an acyl group selected from an optionally substituted C₁-C₅alkylcarbonyl, an optionally substituted C₅-C₁₄arylcarbonyl, an optionally substituted C₅-C₁₄aryl C₁-C₅alkylcarbonyl, an optionally substituted 6 to 10 membered heteroarylcarbonyl, and an optionally substituted 6 to 10 membered heteroaryl-C₁-C₅alkylcarbonyl, C₆-C₁₄aryl-C₁-C₄alkyl, or heteroaryl-C₁-C₄alkyl; or R¹ and R², taken together with the nitrogen, form a 3 to 7 membered non-aromatic heterocyclic ring which may be substituted or may contain one or more additional heteroatoms; R³ =H; and R⁴, R⁵, R⁶, R⁷, R⁸, R⁹ and R¹⁰ are as defined above for general formula **I,** the 3-azabicyclo[3.2.2]nonane derivatives with the general formula **I** can be synthesized from the above mentioned compounds **III, IV, V** or **VIII** by oxidation of one alkyl residue for example with potassium permanganate in acetone and subsequent hydrolysis of the amide **X** to compound **XI,** subsequent N-alkylation or N-acylation to **XII** and further transformation by a Schmidt reaction and reduction to **I** as described above.

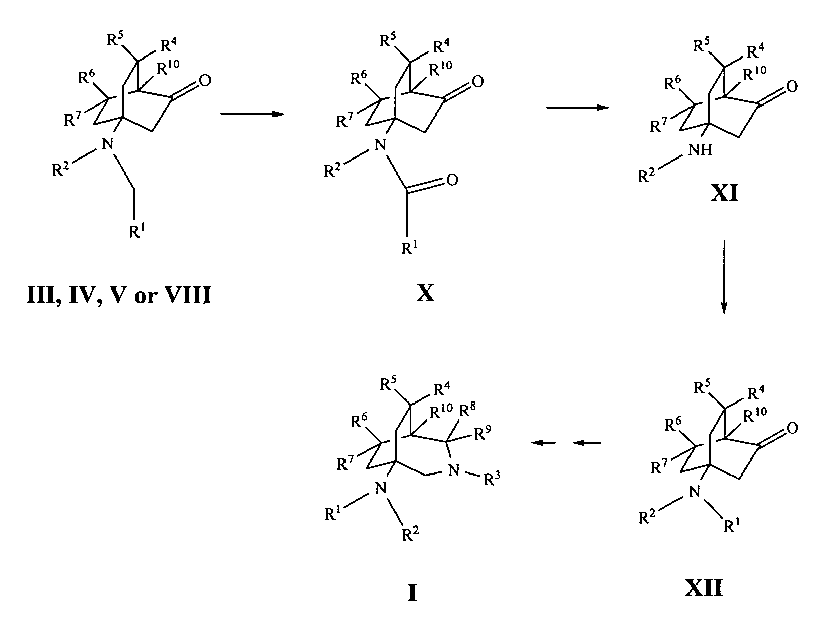

The compounds **I** with R³ ≠ H can be prepared from compounds **I** with R³ = H via N-acylation, N-alkylation or by N-acylation and subsequent reduction.

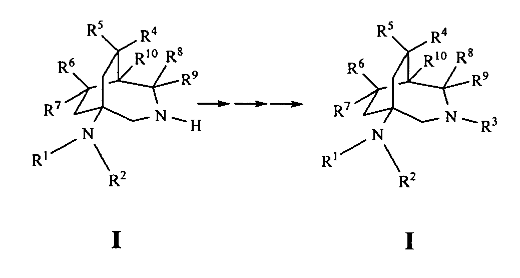

In order to obtain a compound of formula **I,** in which R⁸ and R⁹ taken together form a double bonded oxygen, the last step in preparing a compound of formula **I** as described above in *methods 1 to 4,* namely the reduction step, is omitted. Accordingly, such compounds, in which R⁸ and R⁹ taken together form a double bonded oxygen, (e.g. compounds of formula **II, VI** or **IX)** and which compounds are produced by omitting the reduction step are also compounds of formula **I.**

In the following examples *in vitro* and *in vivo* test methods as well as results are given:

### Test methods:

### Test method for in vitro screening against Plasmodium falciparum

Antiplasmodial activity is determined using the K₁ strain of P. falciparum (resistant to chloroquine and pyrimethamine). A modification of the [³H]-hypoxanthine incorporation assay is used [Matile H, Pink JRL. in: Lefkovits I. and Pernis B. (Eds.). Immunological Methods, Academic Press, San Diego, 1990, 221-234.]. Briefly, infected human red blood cells in RPMI 1640 medium with 5% Albumax are exposed to serial drug dilutions in microtiter plates for 48 hours. Viability is assessed by measuring the incorporation of [³H]-hypoxanthine by liquid scintillation counting 24 hours after the addition of the radiolabel. The counts are expressed as percentage of the control cultures, sigmoidal inhibition curves are drawn and IC₅₀ values calculated.

### Test method for in vitro screening against Trypanosoma brucei rhodesiense and Cytotoxicity

Minimum Essential Medium (50 µl) supplemented according to Baltz et al. [Baltz T, Baltz D, Giroud C, Crockett J. EMBO J., 1985, 4: 1273-1277] with 2-mercaptoethanol and 15% heat-inactivated horse serum is added to each well of a 96-well microtiter plate. Serial drug dilutions are added to the wells. Then 50 µl of trypanosome suspension (T. b. rhodesiense STIB 900) is added to each well and the plate incubated at 37°C under a 5% CO₂ atmosphere for 72 hours. Alamar Blue (10 µl) is then added to each well and incubation continued for a further 2-4 hours. The plate is then read with a Millipore Cytofluor 2300 using an excitation wavelength of 530 nm and emission wavelength of 590 nm [Räz B, Iten M, Grether-Bühler Y, Kaminsky R, Brun R. Acta Trop., 1997, 68: 139-147]. Fluorescence development is expressed as percentage of the control, and IC₅₀ values determined. Cytotoxicity is assessed using the same assay and L-6 cells.

### Test method for in vivo screening against Plasmodium berghei

Method of infection of animals:
   Male mice (Fü albino; specific pathogen free) weighing 20± 2g are infected intravenously with 2x10⁷ P. berghei ANKA strain-infected erythrocytes from donor mice on day 0 of the experiment. Heparinized blood is taken from donor mice with approximately 30% parasitemia and is diluted in physiological saline to 10⁸ parasitized erythrocytes/ml. An aliquot (0.2 ml) of this suspension is injected intravenously into experimental groups of 3 mice and a control group of 5 mice.
   In untreated control mice, parasitemias rise regularly to 30-40% by day +3 after infection and to 70-80% by day +4. The mice die between days +5 and +7 after infection.
Administration of compounds :
   Compounds are prepared at appropriate concentrations, either as solutions or as suspensions containing 3% ethanol and 7% Tween 80. They are administered either subcutaneously or per os in a total volume of 0.01 ml per g of body weight on day +1 (24 hours after infection) of the experiment.
Measuring parasite growth inhibition and survival time:
   On day +3 (48 hours after treatment) blood smears of all animals are prepared and stained with Giemsa. Parasitemia is determined microscopically by counting 1000 red blood cells.
   For low parasitemias (<1%) 2000 rbc's have to be counted. The difference between the mean value for the control group (taken as 100%) and that for each experimental group is calculated and expressed as percent reduction (=activity).
   The mean survival day (MSD, in days) is recorded as well as observations concerning side effects of the drug.

### Test method for in vivo screening against Trypanosoma b. rhodesiense STIB 900; Acute mouse model:

Method of infection of animals:
Females mice (NMRI), 4 mice per group, weighing 20-25g are infected i.p. with 1x10⁵ bloodstream forms of Trypanosoma brucei rhodesiense STIB 900, a clone of a population isolated in 1982 from a patient in Tanzania which is susceptible to all drugs in use. These bloodstream forms come from a stock of cryopreserved stabilates containing 10% glycerol. The stabilate is suspended in PSG (phosphate-saline-glucose) 6:4 [Lanham & Godfrey, Exp. Parasitol. 1970, 28: 521-534] to obtain a trypanosome concentration of 4x10⁵/ml. Each mouse is injected with 0.25 ml. Untreated control mice normally die between day 7-9 post infection.
Administration of compounds
Compounds are prepared at appropriate concentrations in 100% DMSO and further diluted in distilled H₂0, unless another solvent is recommended by the supplier.
They are daily administered i.p. or p.o. in a total volume of 0.01 ml per g of body weight from day +3 to day +6 of the experiment at 50% of the highest tolerated dose (HTD). The HTD is assessed by injecting mice i.p. with increasing doses starting at 5 mg/kg until toxic symptoms are observed.
Measuring parasite growth inhibition and survival time:
On day +5, day +7 and then twice a week until day +60, parasitemia of all animals is checked by tail blood examination and recorded based on a subjective scale ranging from (+) = 1 trypanosome/20 fields to +++ = >100 trypanosomes/field with 20x objective. For mice dying before day +60 the day of death is recorded.
At day +60, surviving and aparasitaemic mice are considered cured and then euthanized.
Results:

| | | |
|---|---|---|
| Preliminary Screen: | | |
| | 4 days at 50% HTD on days +3 to +6 post infection. | |
| | | |
| Activity criteria: | | |
| | Not active: | parasitaemia remains, survival <15 days |
| | Moderately active: | parasitaemia disappears temporarily, survival >20 days |
| | Active: | parasitaemia disappears, survival >60 days |
| | | |
| Dose ranging test: | | |
| | The minimum curative dose is determined by lowering the daily doses of the 4 day treatment schedule. Size of the groups and activity criteria are the same as above. | |

In the case of healing the following chronic model will be applied:

### Test method for in vivo screening against Trypanosoma b. brucei GVR 35 Chronic CNS mouse model

Method of infection of animals :
Females mice (NMRI), 5 mice per group, weighing 20-25g are infected i.p. with 2x10⁴ bloodstream forms of Trypanosoma b. brucei GVR 35 which leads to a CNS infection by day 21 post infection. These bloodstream forms come from a stock of cryopreserved stabilates containing 10% glycerol. The stabilate is suspended in PSG (phosphate-saline-glucose) 6:4 [Lanham & Godfrey, Exp. Parasitol. 1970, 28: 521-534] to obtain a trypanosome concentration of 4x10⁴/ml. Each mouse is injected with 0.25 ml.
Administration of compounds
Compounds are prepared at appropriate concentrations in 100% DMSO and further diluted in distilled H₂0, unless another solvent is recommended by the supplier.
They are daily administered i.p. or p.o. in a total volume of 0.01 ml per g of body weight from day +21 to day +25 of the experiment at 50% of the highest tolerated dose (HTD). The HTD is assessed by injecting mice i.p. with increasing doses starting at 5 mg/kg until toxic symptoms are observed.
Measuring parasite growth inhibition and survival time:
On day +28 and then twice a week until day +50 parasitemia of all animals is checked by tail blood examination and recorded based on a subjective scale ranging from (+) = 1 trypanosome/20 fields to +++ = >100 trypanosomes/field with 20x objective. After day +50 the mice are checked only once per week until day +180. For mice dying before day +180 the day of death is recorded.
At day +180, surviving and aparasitaemic mice are considered cured and then euthanized.
Results:

| | | |
|---|---|---|
| Activity criteria: | | |
| | Not active: | survival <10 days longer than the MSD for the diminazene control |
| | Moderately active: | survival >10 days longer than the MSD for the diminazene control but relapse before day +180 |
| | Active: | >80% of the animals aparasitaemic until day +180 |

The activities of the compounds with the general formula I are documented by the activities of example 1 ((6RS,9RS)-(±)-6,9-Diphenyl-1-pyrrolidino-3-azabicyclo[3.2.2]nonane):
In vitro activity

| Example | IC₅₀ P.falc. µM | IC₅₀ T.rho. µM | IC₅₀ Cytox µM |
|---|---|---|---|
| 1 | 0.095 | 1.00 | 72.93 |
| Chloroquine | 0.12 | - | 188.5 |
| Artemisinin | 0.0064 | - | 450.5 |
| Melarsoprol | - | 0.0039 | 7.78 |
| Pentamidine | - | 0.0075 | 4724.5 |

In vivo activity

| Example | Appl./dose | MSD P. berghei | Activity % | control |
|---|---|---|---|---|
| 1 | ip 4x50mg/kg | 12.3 | 94.32 | 4 |
| 1 | ip 4x10mg/kg | 4 | 25.06 | 4 |
| 1 | ip 4x20mg/kg | 10 | 60.11 | 4 |
| 1 | ip 4x50mg/kg | 15 | 94.77 | 4 |
| 1 | po 4x100mg/kg | 18.6 | 97.7 | 4 |
| 1 | po 1x100mg/kg | 12 | 70.52 | 3 |
| 1 | po 1x200mg/kg | 16, toxic | 66.13 | 3 |
| Chloroquine | ip 4x10mg/kg | 20 | 99.6 | 4 |
| Chloroquine | po 4x10mg/kg | 20 | 99.6 | 4 |

The present invention is further demonstrated and illustrated by the following examples, yet without being restricted thereto.

### Example 1:

### (6RS,9RS)-(±)-6,9-Diphenyl-1-pyrrolidino-3-azabicyclo[3.2.2]nonane

### (6RS,7RS)-(±)-6,7-Diphenyl-4-pyrrolidinobicyclo[2.2.2]octan-2-one x HSCN

In a round-bottomed flask pyrrolidinium thiocyanate (13 g, 0.1 mol) and benzylidene acetone (29.2 g 0.2 mol) are suspended in dimethylformamide (120 ml). The mixture is refluxed at a water separator for 6 hours and thereupon the solvent removed in vacuo. The residue is triturated with ethanol, filtered with suction and recrystallized from ethanol yielding 28.9 g (71 %) white crystals, M.p.: 202°C.

### (7RS,8RS)-(±)-7,8-Diphenyl-5-pyrrolidino-3-azabicyclo[3.2.2]nonan-2-one

The hydrothiocyanate of (6RS,7RS)-(±)-6,7-diphenyl-4-pyrrolidinobicyclo[2.2.2]octan-2-one (2 g, 4.94 mmol) is dissolved in concentrated sulfuric acid (16 ml) under stirring and cooling on an ice bath. The solution is allowed to warm up to room temperature and NaN₃ (500 mg, 7.7 mmol) is added in portions. After the addition is completed, the solution is poored on ice, alkalized with 2M NaOH and extracted 5 times with CH₂Cl₂. The combined organic phases are washed 3 times with water, dried over sodium sulfate, filtered and the solvent is evaporated in vacuo giving a yellow resin (1.39 g, 78%) pure enough for further reactions. It can be crystallized from ethanol/water. M.p.: 161°C;
¹H NMR (CDCl₃, δ, 400 MHz): 1.77 (br, s, 4H), 2.16 - 2.28 (m, 4H), 2.70 - 2.77 (m, 4H), 2.88 (s, 1H), 3.39 - 3.49 (m, 4H), 5.84 (d, *J* = 2.8 Hz, 1H), 7.21 - 7.38 (m, 10H) ppm.

### (6RS,9RS)-(±)-6,9-Diphenyl-1-pyrrolidino-3-azabicyclo[3.2.2]nonane

The (7RS,8RS)-(±)-7,8-diphenyl-5-pyrrolidino-3-azabicyclo[3.2.2]nonan-2-one (2.15 g, 6.0 mmol) is suspended in dry ether (100 ml) with stirring and cooling and powdered LiAlH₄ (1.2 g, 31.6 mmol) is added in portions. Then the suspension is refluxed over night cooled to room temperature and quenched with water cautiously under stirring and cooling on an ice bath. 2N NaOH is added and the mixture is extracted five times with ether, washed twice with water, dried (Na₂SO₄) and filtered. The solvent is evaporated in vacuo giving a resinous residue (1.91 g, 92%). The hydrochloride is prepared by repeated treatment of solutions of the base with 2M ethereal HCl, evaporation of the solvent and crystallization from ethanol/acetone. M.p. (HCl): 205°C;
¹H NMR (CDCl₃, δ, 400 MHz): 1.74 - 1.77 (m, 4H), 2.01 (br, s, 1H), 2.06 (t, *J* = 12.8 Hz, 1H), 2.07 (t, *J* = 12.8 Hz, 1H), 2.29 (ddd, *J* = 12.8, 8.8, 2.2 Hz, 1H), 2.49 (ddd, *J* = 12.8, 9.3, 2.4 Hz, 1H), 2.71 - 2.79 (m, 4H), 2.88 (br, d, *J* = 12.1 Hz, 1H), 3.12 (dd, *J* = 12.8, 4.2 Hz, 1H), 3.15 (s, 2H), 3.70 (br, t, *J* = 9.5 Hz, 2H), 7.16 - 7.41 (m, 10H) ppm.

### Example 2:

### (6RS,9RS)-(±)-1-Dimethylamino-6,9-diphenyl-3-azabicyclo[3.2.2]nonane

### (6RS,7RS)-(±)-4-Dimethylamino-6,7-diphenylbicyclo[2.2.2]octan-2-one x HSCN

Dimethylammonium thiocyanate (10.4 g, 0.1 mol) and benzylidene acetone (29.2 g, 0.2 mol) give 32.7 g (86 %) white crystals, M.p.:196°C.

### (7RS,8RS)-(±)-5-Dimethylamino-7,8-diphenyl-3-azabicyclo[3.2.2]nonan-2-one

The hydrothiocyanate of (6RS,7RS)-(±)-4-dimethylamino-6,7-diphenylbicyclo[2.2.2]octan-2-one (4 g, 10.6 mmol) reacts with NaN₃ (1 g, 15.4 mmol) in concentrated sulfuric acid (32 ml) to a yellow resin (3.35 g 80%) pure enough for further reactions. It can be crystallized from ethanol/water. M.p. : 162°C.
¹H NMR (CDCl₃, δ, 400 MHz): 2.06 -2.12 (m, 2H), 2.18 - 2.26 (m, 2H), 2.31 (s, 6H), 2.86 (br, s, 1H), 3.35 (d, *J* =1.8 Hz, 2H), 3.36 - 3.45 (m, 2H), 6.43 (br, s, 1H), 7.17 - 7.38 (m, 10H) ppm.

### (6RS,9RS)-(±)-1-Dimethylamino-6,9-diphenyl-3-azabicyclo[3.2.2]nonane

The (7RS,8RS)-(±)-5-dimethylamino-7,8-diphenyl-3-azabicyclo[3.2.2]nonan-2-one (1 g, 3.00 mmol) gives with LiAlH₄ (570 mg, 15.00 mmol) in dry ether (50 ml) a resinous residue (928 mg, 97%). The hydrochloride crystallized from ethanol/ethyl acetate. M.p. (HCl): 244°C.
¹H NMR (CDCl₃, δ*,* 400 MHz): 1.89 -1.97 (m, 2H), 1.99 (br, s, 1H), 2.28 (ddd, *J* = 12.8, 8.8, 2.6 Hz, 1H), 2.30 (s, 6H), 2.46 (ddd, *J* = 12.3, 6.7, 2.4 Hz, 1H), 2.88 (d, *J* = 12.5 Hz, 1H), 3.05 (d, *J* = 13.6 Hz, 1H), 3.12 (d, *J* = 12.8 Hz, 1H), 3.13 (d, *J* = 12.8 Hz, 1H), 3.30 - 3.37 (m, 2H), 7.16 - 7.40 (m, 10H) ppm.

### Example 3:

### (6RS,9RS)-(±)-1-Morpholino-6,9-diphenyl-3-azabicyclo[3.2.2]nonane

### (6RS,7RS)-(±)-4-Morpholino-6,7-diphenylbicyclo[2.2.2]octan-2-one x HSCN

Morpholinium thiocyanate (14.6 g, 0.1 mol) and benzylidene acetone (29.24 g, 0.2 mol) give 35.3 g (84 %) white crystals. M.p. 208°C.

### (7RS,8RS)-(±)-5-Morpholino-7,8-diphenyl-3-azabicyclo[3.2.2]nonan-2-one

The hydrothiocyanate of (6RS,7RS)-(±)-4-Morpholino-6,7-diphenylbicyclo[2.2.2]octan-2-one (2 g, 4.76 mmol) gives with NaN₃ (500 mg, 7.7 mmol) in concentrated sulfuric acid (16 ml) a yellow resin (1.70 g 95%) pure enough for further reactions. It can be crystallized from ethanol. M.p.:197°C.
¹H NMR (CDCl₃, δ, 400 MHz): 2.04 (t, *J* = 12.1 Hz, 1H), 2.09 (t, *J* = 12.1 Hz, 1H), 2.17 - 2.22 (m, 2H), 2.54 - 2.70 (m, 4H), 2.88 (br, s, 1H), 3.33 (br, s, 2H), 3.41 (br, t, *J* = 9.7 Hz, 1H), 3.42 (br, t, *J* = 9.5 Hz, 1H), 3.66 - 3.71 (m, 4H), 6.70 (br, s, 1H), 7.19 - 7.37 (m, 10H) ppm.

### (6RS,9RS)-(±)-1-Morpholino-6,9-diphenyl-3-azabicyclo[3.2.2]nonane

The (7RS,8RS)-(±)-5-Morpholino-7,8-diphenyl-3-azabicyclo[3.2.2]nonan-2-one (1 g, 2.66 mmol) gives with LiAlH₄ (600 mg, 15.81 mmol) in dry ether (50 ml) a resinous residue (840 mg, 87%). The hydrochloride crystallized from ethyl acetate. M.p. (HCl): 235°C.
¹H NMR (CDCl₃, δ, 400 MHz): 1.88 (t, *J* = 11.5 Hz, 1H), 1.95 (dd, *J* = 12.5, 10.3 Hz, 1H), 2.02 (br, s, 1H), 2.23 (ddd, *J* = 12.1, 9.0, 2.4 Hz, 1H), 2.46 (ddd, *J* = 12.1, 9.2, 2.2 Hz, 1H), 2.58 - 2.72 (m, 4H), 2.88 (d, *J* = 12.5 Hz, 1H), 3.06 (d, *J* = 13.6 Hz, 1H), 3.12 (dd, *J* = 12.8 Hz, 1H), 3.13 (d, *J* = 12.8 Hz, 1H), 3.34 (br, t, *J* = 9.5 Hz, 2H) 3.68 - 3.73 (m, 4H), 7.17 - 7.40 (m, 10H) ppm.

### Example 4:

### (6RS,9RS)-(±)-6,9-Diphenyl-1-piperidino-3-azabicyclo[3.2.2]nonane

### (6RS,7RS)-(±)-6,7-Diphenyl-4-piperidinobicyclo[2.2.2]octan-2-one x HSCN

Piperidinium thiocyanate (39 g, 0.27 mol) and benzylidene acetone (67.4 g, 0.46 mol) give 15.9 g (17%) white crystals. M.p.:264°C

### (7RS,8RS)-(±)-7,8-Diphenyl-5-piperidino-3-azabicyclo[3.2.2]nonan-2-one

The hydrothiocyanate of (6RS,7RS)-(±)-6,7-diphenyl-4-piperidinobicyclo[2.2.2]octan-2-one (3 g, 7.17 mmol) gives with NaN₃ (750 mg, 11.5 mmol) in concentrated sulfuric acid (24 ml) a yellow resin (2.3 g, 86%) pure enough for further reactions. It can be crystallized from ethanol. M.p.: 216°C.
¹H NMR (CDCl₃, δ, 400 MHz): 1.40 - 1.48 (m, 2H), 1.50 - 1.62 (m, 4H), 2.05 (t, *J* = 12.5 Hz, 1H), 2.12 - 2.28 (m, 3H), 2.46 - 2.66 (m, 4H), 2.86 (s, 1H), 3.33 (s, 2H), 3.36 - 3.42 (m, 2H), 6.69 (s, 1H), 7.17 - 7.38 (m, 10H) ppm.

### (6RS,9RS)-(±)-6,9-Diphenyl-1-piperidino-3-azabicyclo[3.2.2]nonane

The (7RS,8RS)-(±)-7,8-diphenyl-5-piperidino-3-azabicyclo[3.2.2]nonan-2-one (1.45 g, 3.9 mmol) gives with LiAlH₄ (970 mg, 25.6 mmol) in dry ether (75 ml) a resinous residue (1.25 g, 88%). The hydrochloride is crystallized from ethyl acetate. M.p. (HCl): 230°C.
¹H NMR (CDCl₃, δ, 400 MHz): 1.42 - 1.46 (m, 2H), 1.54 - 1.60 (m, 4H), 1.87 - 2.01 (m, 3H), 2.25 (ddd, *J* =11.7, 9.8, 1.8 Hz, 1H), 2.44 (ddd, *J* = 11.9, 10.3,1.8 Hz, 1H), 2.50 - 2.68 (m, 4H), 2.87 (d, *J* = 12.8 Hz, 1H), 3.05 (d, *J* = 13.6 Hz, 1H), 3.09 - 3.13 (m, 2H), 3.32 (br, t, *J* = 9.5 Hz, 2H), 7.16 -7.39 (m, 10 H) ppm.

### Example 5:

### (6RS,9RS)-(±)-1-(4-Methylpiperazin-1-yl)-6,9-diphenyl-3-azabicyclo[3.2.2]nonane

### (6RS,7RS)-(±)-4-(4-Methylpiperazin-1-yl)-6,7-diphenylbicyclo[2.2.2]octan-2-one x HSCN

N-Methyl-piperazinium thiocyanate and benzylidene acetone give after reaction and work-up white crystals.

### (7RS,8RS)-(±)-7,8-Diphenyl-5-(4-methylpiperazin-1-yl)-3-azabicyclo[3.2.2]nonan-2-one

The hydrothiocyanate of (6RS,7RS)-(±)-4-(4-methylpiperazin-1-yl)-6,7-diphenylbicyclo[2.2.2]octan-2-one (3.03 g, 7 mmol) gives with NaN₃ (658 mg, 10.1 mmol) in concentrated sulfuric acid (21 ml) a resin (2.7 g, 99%), which crystallizes from acetone/ethanol. M.p. 243 °C.
¹H NMR (CDCl₃, δ, 400 MHz): 2.06 (t, *J* = 11.9 Hz, 1H), 2.11 - 2.23 (m, 3H), 2.26 (s, 3H), 2.42 (s, 4H), 2.64 (s, 2H), 2.72 (s, 2H), 2.87 (s, 1H), 3.33 (d, *J* = 1.83 Hz, 2H), 3.37 - 3.43 (m, 2H), 6.57 (s, 1H), 7.18 - 7.37 (m, 10H) ppm.

### (6RS,9RS)-(±)-1-(4-Methylpiperazin-1-yl)-6,9-diphenyl-3-azabicyclo[3.2.2]nonane

The (7RS,8RS)-(±)-7,8-diphenyl-5-(4-methylpiperazin-1-yl)-3-azabicyclo[3.2.2]nonane (1.7 g, 4.3 mmol) gives with LiAlH₄ (822 mg, 21.6 mmol) in dry ether (73 ml) a resin (527 mg, 32%), which is transformed to its hydrochloride and crystallized from methanol/water. M.p. (HCl): 300°C.
¹H NMR (CDCl₃, δ, 400 MHz): 1.89 (t, *J* = 11.7 Hz, 1H),1.96 (ddd, *J* =12.5, 10.3, 2.2 Hz, 1H), 2.01 (s, 1H), 2.24 - 2.30 (m, 4H), 2.34 - 2.54 (m, 5H), 2.62 - 2.78 (m, 4H), 2.88 (d, *J* = 12.8 Hz, 1H), 3.11 (d, *J* = 13.6 Hz, 1H), 3.10 - 3.16 (m, 2H), 3.34 (br, t, *J* = 9.3 Hz, 2H), 7.16 -7.40 (m, 10H) ppm.

### Example 6:

### (6RS,9RS)-(±)-6,9-Bis(4-chlorophenyl)-1-dimethylamino-3-azabicyclo[3.2.2]nonane

### (6RS,7RS)-(±)-6,7-Bis(4-chlorophenyl)-4-dimethylaminobicyclo[2.2.2]octan-2-one x HSCN

Dimethylammonium thiocyanate (17.70 g, 0.17 mmol) and 4-(4-chlorophenyl)-but-3-en-2-one (30.8g, 0.17mmol) give after reaction and work-up crystals (10.4 g,13.6%).

### (7RS,8RS)-(±)-7,8-Bis(4-chlorophenyl)-5-dimethylamino-3-azabicyclo[3.2.2]nonan-2-one

The hydrothiocyanate of (6RS,7RS)-(±)-6,7-bis(4-chlorophenyl)-4-dimethylaminobicyclo[2.2.2]octan-2-one (4.74g, 10.6 mmol) gives with NaN₃ (1.00 g, 15.4 mmol) in concentrated sulfuric acid (32 ml) a residue which gives a yellow precipitate (3.04 g, 71 %) after treatment with hexane.
¹H NMR (CDCl₃, δ, 400 MHz): 1.98 -2.06 (m, 2H), 2.16 - 2.56 (m, 2H), 2.30 (s, 6H), 2.73 (br, s, 1H), 3.29 (ddd, *J* = 11.4, 8.8, 2.6 Hz, 1H), 3.33 (br, s, 2H), 3.39 (dd, *J* = 11.4, 8.1 Hz, 1H), 6.84 (br, s, 1H), 7.22 - 7.34 (m, 8H) ppm.

### (6RS,9RS)-(±)-6,9-Bis(4-chlorophenyl)-1-dimethylamino-3-azabicyclo[3.2.2]nonane

The (7RS,8RS)-(±)-7,8-bis(4-chlorophenyl)-5-dimethylamino-3-azabicyclo[3.2.2]nonan-2-one (2.44 g, 6.05 mmol) gives with LiAlH₄ (1.15 g, 30.3 mmol) in dry ether (100 ml) a colourless resin (2.02 g, 86%). The hydrochloride crystallized from ethyl acetate or ethanol/ethyl acetate. M.p. (HCl): 255°C.
¹H NMR (CDCl₃, δ, 400 MHz): 1.84 - 1.95 (m, 3H), 2.25 -2.36 (m, 7H), 2.45 (br, dd, *J* = 11.7, 10.6 Hz, 1H), 2.87 (d, *J* = 12.5 Hz, 1H), 3.01 - 3.14 (m, 3H), 3.20 (ddd, *J* = 12.1, 9.5, 2.6 Hz, 1H), 3.31 (t, *J* = 9.5 Hz, 1H), 7.23 - 7.35 (m, 8H) ppm.

### Example 7:

### (6RS,9RS)-(±)-6,9-Bis(4-chlorophenyl)-1-pyrrohdino-3-azabicyclo[3.2.2]nonane

### (6RS,7RS)-(±)-6,7-Bis(4-chlorophenyl)-4-pyrrolidinobicyclo[2.2.2]octan-2-one x HSCN

Pyrrolidinium thiocyanate (52 g, 0.4 mol) and 4-(4-chlorophenyl)-but-3-en-2-one (82 g, 0.45 mol) give after reaction and work-up yellow crystals (6.2 g, 2.6%).

### (7RS,8RS)-(±)-7,8-Bis(4-chlorophenyl)-5-pyrrolidino-3-azabicyclo[3.2.2]nonan-2-one

The hydrothiocyanate of (6RS,7RS)-(±)-6,7-bis(4-chlorophenyl)-4-pyrrolidinobicyclo[2.2.2]octan-2-one (5.02 g, 10.6 mmol) gives with NaN₃ (1.00 g, 15.4 mmol) in concentrated sulfuric acid (32 ml) a precipitate (4.34 g, 95%) after treatment with ethanol. ¹H NMR (CDCl₃, δ, 400 MHz): 1.77 (br, s, 4H), 2.06 - 2.15 (m, 2H), 2.20 - 2.29 (m, 2H), 2.68 - 2.76 (m, 4H), 2.76 (s, 1H), 3.31 (ddd, *J* = 11.4, 8.8, 2,6 Hz, 1H), 3.37 - 3.46 (m, 3H), 6.09 (br, s, 1H), 7.21 - 7.34 (m, 8H) ppm.

### (6RS,9RS)-(±)-6,9-Bis(4-chlorophenyl)-1-pyrrolidino-3-azabicyclo[3.2.2]nonane

The (7RS,8RS)-(±)-7,8-bis(4-chlorophenyl)-5-pyrrolidino-3-azabicyclo[3.2.2]nonan-2-one (2.37 g, 5.52 mmol) gives with LiAlH₄ (1.05 g, 27.7 mmol) in dry ether (90 ml) a colourless resin (1.18 g, 51.5%). The hydrochloride crystallized from ethyl acetate or ethanol/ethyl acetate. M.p. (HCl): 244°C.
¹H NMR (CDCl₃, δ, 400 MHz): 1.58 (br, s, 1H), 1.72 - 1.78 (m, 4H), 1.86 (br, s, 1H), 1.93 - 2.03 (m, 2H), 2.28 (ddd, *J* = 12.1, 9.0, 2.2 Hz, 1H), 2.47 (ddd, *J* = 12.1, 9.2, 2.2 Hz, 1H), 2.64 - 2.80 (m, 4H), 2.88 (d, *J* = 12.5 Hz, 1H), 3.06 (dd, *J* = 12.5, 4.4 Hz, 1H), 3.08 - 3.18 (m, 2H), 3.22 (ddd, *J* = 12.5, 9.9, 3.3 Hz, 1H), 3.33 (br, t, *J* = 9.5 Hz, 1H), 7.23 - 7.33 (m, 8H) ppm.

### Example 8:

### (6RS,9RS)-(±)-6,9-Bis(4-chlorophenyl)-1-piperidino-3-azabicyclo[3.2.2]nonane

### (6RS,7RS)-(±)-6,7-Bis(4-chlorophenyl)-4-piperidinobicyclo[2.2.2]octan-2-one x HSCN

Piperidinium thiocyanate (39.23 g, 0.27mol) and 4-(4-chlorophenyl)-but-3-en-2-one (49.37g, 0.27mol) yield crystals (10.2 g, 27%) after reaction and work-up.

### (7RS,8RS)-(±)-7,8-Bis(4-chlorophenyl)-5-piperidino-3-azabicyclo[3.2.2]nonan-2-one

The hydrothiocyanate (5.17 g, 10.6 mmol) of (6RS,7RS)-(±)-6,7-bis(4-chlorophenyl)-4-piperidinobicyclo[2.2.2]octan-2-one gives with NaN₃ (1.00 g, 15.4 mmol) in concentrated sulfuric acid (32 ml) a residue giving a precipitate (4.63 g, 99%) after treatment with ethanol. ¹H NMR (CDCl₃, δ, 400 MHz): 1.44 - 1.48 (m, 2H), 1.54 - 1.62 (m, 4H), 1.97 - 2.09 (m, 2H), 2.17 - 2.28 (m, 2H), 2.50 - 2.68 (m, 4H), 2.76 (s, 1H), 3.28 (ddd, *J* =11.0, 8.4, 2.6 Hz, 1H), 3.35 - 3.90 (m, 3H), 5.90 (s, 1H), 7.20 - 7.33 (m, 8H) ppm.

### (6RS,9RS)-(±)-6,9-Bis(4-chlorophenyl)-1-pyrrolidino-3-azabicyclo[3.2.2]nonane

The (7RS,8RS)-(±)-7,8-bis(4-chlorophenyl)-5-piperidino-3-azabicyclo[3.2.2]nonan-2-one (2.23 g, 5.03 mmol) gives with LiAlH₄ (960 mg, 25.3 mmol) in dry ether (85 ml) a colourless resin (1.67 g, 77.3%). The hydrochloride crystallizes from ethyl acetate or ethanol/ethyl acetate. M.p. (HCl, decomp.): 262°C.
¹H NMR (CDCl₃, δ, 400 MHz): 1.42 - 1.48 (m, 2H), 1.52 - 1.64 (m, 4H), 1.83 (t, *J* = 11.7 Hz, 1H), 1.86 (br, s, 1H), 1.91 (dd, *J* = 12.8, 10.3 Hz, 1H), 2.25 (ddd, *J* = 12.5, 8.8, 1.8 Hz, 1H), 2.45 (ddd, *J* = 11.9, 10.1, 1.8 Hz, 1H), 2.49 - 2.68 (m, 4H), 2.87 (d, *J* = 12.5 Hz, 1H), 3.02 (d, *J* = 13.6 Hz, 1H), 3.07 (dd, *J* = 12.5, 4.4 Hz, 1H), 3.12 (d, *J* = 13.6 Hz, 1H), 3.19 (ddd, *J* = 12.5, 9.5, 2.9 Hz, 1H), 3.29 (br, t, *J* = 9.5 Hz, 1H), 7.23 - 7.34 (m, 8H) ppm.

### Example 9:

### (6RS,9RS)-(±)-1-Dimethylamino-6,9-bis(4-methylphenyl)-3-azabicyclo[3.2.2]nonane

### (6RS,7RS)-(±)-4-Dimethylamino-6,7-bis(4-methylphenyl)bicyclo[2.2.2]octan-2-one x HSCN

Dimethylammonium thiocyanate (5.73 g, 55 mmol) and 4-(4-methylphenyl)but-3-en-2-one (17.63 g,110 mmol) give crystals (6.92 g, 31.0 %).

### (7RS,8RS)-(±)-5-Dimethylamino-7,8-bis(4-methylphenyl)-3-azabicyclo[3.2.2]nonan-2-one

The hydrothiocyanate of (6RS,7RS)-(±)-4-dimethylamino-6,7-bis(4-methylphenyl)bicyclo[2.2.2]octan-2-one (4.31 g, 10.6 mmol) gives with NaN₃ (1.00 g, 15.4 mmol) in concentrated sulfuric acid (32 ml) a resin (476 mg,12%).

### (6RS,9RS)-(±)-1-Dimethylamino-6,9-bis(4-methylphenyl)-3-azabicyclo[3.2.2]nonane

The (7RS,8RS)-(±)-7,8-bis(4-methylphenyl)-5-dimethylamino-3-azabicyclo[3.2.2]nonan-2-one (473 mg, 1.30 mmol) gives with LiAlH₄ (248 mg, 6.52 mmol) in dry ether (22 ml) a colourless resin (354 mg, 78.1 %). The hydrochloride crystallizes from acetone. M.p. (HCl): 266°C.
¹H NMR (CDCl₃, δ, 400 MHz): 1.86 - 1.98 (m, 3H), 2.26 (ddd, *J* = 12.5, 8.9, 2.2 Hz, 1H), 2.31, 2.34 (2s, 12H), 2.41 (ddd, *J* = 12.5, 9.2, 2.2 Hz, 1H), 2.87 (d, *J* = 13.2 Hz, 1H), 3.05 (d, *J* = 13.6 Hz, 1H), 3.11 (d, *J* = 13.6 Hz, 1H), 3.12 (dd, *J* = 12.8, 4.4 Hz, 1H), 3.26 - 3.33 (m, 2H), 7.10 - 7.29 (m, 8H) ppm.

### Example 10:

### (6RS,9RS)-(±)-6,9-Bis(4-methylphenyl)-1-pyrrolidino-3-azabicyclo[3.2.2]nonane

### (6RS,7RS)-(±)-6,7-Bis(4-methylphenyl)-4-pyrrolidinobicyclo[2.2.2]octan-2-one x HSCN

Pyrrolidinium thiocyanate (11.03 g, 85 mmol) and 4-(4-methylphenyl)but-3-en-2-one (27.14 g, 169 mmol) give crystals (12.72 g, 34.6 %).

### (7RS,8RS)-(±)-7,8-Bis(4-methylphenyl)-5-pyrrolidino-3-azabicyclo[3.2.2]nonan-2-one

The hydrothiocyanate of (6RS,7RS)-(±)-6,7-bis(4-methylphenyl)-4-pyrrolidinobicyclo[2.2.2]octan-2-one (4.59 g, 10.6 mmol) gives with NaN₃ (1 g, 15.4 mmol) in concentrated sulfuric acid (32 ml) a resin (1.41 g, 34%).

### (6RS,9RS)-(±)-6,9-Bis(4-methylphenyl)-1-pyrrolidino-3-azabicyclo[3.2.2]nonane

The (7RS,8RS)-(±)-7,8-bis(4-methylphenyl)-5-pyrrolidino-3-azabicyclo[3.2.2]nonan-2-one (1.37 g, 3.52 mmol) gives with LiAlH₄ (670 mg, 17.65 mmol) in dry ether (60 ml) a colourless resin (640 mg, 49%). The hydrochloride crystallizes from acetone. M.p. (HCl, decomp.): 270 °C.
¹H NMR (CDCl₃, δ, 400 MHz): 1.74 (br, s, 4H), 1.97 - 2.07 (m, 3H), 2.26 (ddd, *J* = 12.8, 9.5, 3.3 Hz, 1H), 2.31, 2.34 (2s, 6H), 2.43 (ddd, t, *J* = 11.9, 10.4, 1.5 Hz, 1H), 2.66 - 2.79 (m, 4H), 2.86 (d, *J* = 13.2 Hz, 1H), 3.10 - 3.13 (m, 3H), 3.32 (br, t, *J* = 9.5 Hz, 2H), 7.09 - 7.29 (m, 8H) ppm.

### Example 11:

### (6RS,9RS)-(±)-6,9-Bis(4-methylphenyl)-1-piperidino-3-azabicyclo[3.2.2]nonane

### (6RS,7RS)-(±)-6,7-Bis(4-methylphenyl)-4-piperidinobicyclo[2.2.2]octan-2-one x HSCN

Piperidinium thiocyanate (7.93 g, 55 mmol) and 4-(4-methylphenyl)but-3-en-2-one (17.63 g, 110 mmol) reacts to crystals (4.09 g,17%).

### (7RS,8RS)-(±)-7,8-Bis(4-methylphenyl)-5-piperidino-3-azabicyclo[3.2.2]nonan-2-one

The hydrothiocyanate of (6RS,7RS)-(±)-6,7-bis(4-methylphenyl)-4-piperidinobicyclo[2.2.2]octan-2-one (4.00 g, 8.96 mmol) gives with NaN₃ (846 mg, 13.0 mmol) in concentrated sulfuric acid (27 ml) a resin (811 mg, 23%).

### (6RS,9RS)-(±)-6,9-Bis(4-methylphenyl)-1-piperidino-3-azabicyclo[3.2.2]nonane

The (7RS,8RS)-(±)-7,8-bis(4-methylphenyl)-5-piperidino-3-azabicyclo[3.2.2]nonan-2-one (593 mg, 1.47 mmol) gives with LiAlH₄ (280 mg, 7.38 mmol) in dry ether (25 ml) a colourless resin (300 mg, 52 %). The hydrochloride crystallizes from acetone. M.p. (HCl, decomp.): 230°C.
¹H NMR (CDCl₃, δ, 400 MHz): 1.40 - 1.48 (m, 2H), 1.50 - 1.64 (m, 4H), 1.88 (br, t, *J* = 11.5 Hz, 1H), 1.94 - 2.00 (m, 2H), 2.23 (br, t, *J* = 11.5 Hz, 1H), 2.31, 2.33 (2s, 6H), 2.40 (br, t, *J* = 11.2 Hz, 1H), 2.52 - 2.68 (m, 4H), 2.87 (d, *J* = 12.8 Hz, 1H), 3.04 - 3.14 (m, 3H), 3.28 (br, t, *J* = 9.3 Hz, 2H), 7.09 - 7.28 (m, 8H) ppm.

### Example 12:

### (6RS,9RS)-(±)-1-Dimethylamino-6,9-bis(2-chlorophenyl)-3-azabicyclo[3.2.2]nonane

### (6RS,7RS)-(±)-4-Dimethylamino-6,7-bis(2-chlorophenyl)bicyclo[2.2.2]octan-2-one x HSCN

Dimethylammonium thiocyanate (17.7 g, 0.17 mol) and 4-(2-chlorophenyl)but-3-en-2-one (61.4 g, 0.34 mol) yield after reaction and work-up crystals (28.3 g, 37%).

### (7RS,8RS)-(±)-7,8-Bis(2-chlorophenyl)-5-dimethylamino-3-azabicyclo[3.2.2]nonan-2-one

The hydrothiocyanate of (6RS,7RS)-(±)-4-dimethylamino-6,7-bis(2-chlorophenyl)bicyclo[2.2.2]octan-2-one (4 g, 8.9 mmol) gives with NaN₃ (845 mg, 13.0 mmol) in concentrated sulfuric acid (27 ml) a resin (3.44 g, 95%).

### (6RS,9RS)-(±)-1-Dimethylamino-6,9-bis(2-chlorophenyl)-3-azabicyclo[3.2.2]nonane

The (7RS,8RS)-(±)-7,8-bis(2-chlorophenyl)-5-dimethylamino-3-azabicyclo[3.2.2]nonan-2-one (1.2 g, 3 mmol) gives with LiAlH₄ (570 mg, 15 mmol) in dry ether (50 ml) a colourless resin (1.09 g, 93%) The hydrochloride crystallizes from 2-propanol. M. p. (HCl): 256°C.
¹H NMR (CDCl₃, δ, 400 MHz): 1.71 (br, s, 1H), 1.89 (dd, *J* = 12.5, 10.3 Hz, 1H), 2.02 (dd, *J* = 12.5, 11.0 Hz, 1H), 2.25 (ddd, *J* = 13.2, 8.8, 2.2 Hz, 1H), 2.33 (s, 6H), 2.54 (ddd, *J* = 12.5, 9.5, 2.2 Hz, 1H), 2.97 (dd, *J* =12.5, 4.4 Hz, 1H), 3.04 (d, *J* = 11.0 Hz, 1H), 3.10 (d, *J* = 12.5 Hz, 1H), 3.17 (d, *J* = 12.5 Hz, 1H), 3.74 (ddd, *J* = 12.5, 9.5, 2.9 Hz, 1H), 3.86 (br, t, *J* = 9.5 Hz, 1H), 7.09 (ddd, *J* = 8.8, 7.3, 1.5 Hz, 1H), 7.16 (ddd, *J* = 9.5, 7.3, 1.5 Hz, 1H), 7.23 -7.30 (m, 3H), 7.38 (br, d, *J* = 8.8 Hz, 1H), 7.75 (br, d, *J* = 7.3 Hz, 1H), 7.94 (br, d, *J* = 8.1 Hz, 1H) ppm.

### Example 13:

### (6RS,9RS)-(±)-6,9-Bis(2-chlorophenyl)-1-pyrrolidino-3-azabicyclo[3.2.2]nonane

### (6RS,7RS)-(±)-6,7-bis(2-chlorophenyl)-4-pyrrolidinobicyclo[2.2.2]octan-2-one x HSCN

Pyrrolidinium thiocyanate (22.1 g, 0.17 mol) and 4-(2-chlorophenyl)but-3-en-2-one (61.4 g, 0.34 mol) give after reaction and work-up crystals (21.89 g, 27.2 %).

### (7RS,8RS)-(±)-7,8-Bis(2-chlorophenyl)-5-pyrrolidino-3-azabicyclo[3.2.2]nonan-2-one

The hydrothiocyanate of (6RS,7RS)-(±)-6,7-bis(2-chlorophenyl)-4-pyrrolidinobicyclo[2.2.2]octan-2-one (4 g, 8.5 mmol) gives with NaN₃ (799 mg, 12.3 mmol) in concentrated sulfuric acid (26 ml) a resin (3.50 g, 96%).

### (6RS,9RS)-(±)-6,9-Bis(2-chlorophenyl)-1-pyrrolidino-3-azabicyclo[3.2.2]nonane

The (7RS,8RS)-(±)-7,8-bis(2-chlorophenyl)-5-pyrrolidino-3-azabicyclo[3.2.2]nonan-2-one (371 mg, 0.86 mmol) gives with LiAlH₄ (164 mg, 4.32 mmol) in dry ether (15 ml) a colourless resin (287 mg, 80.0 %). The hydrochloride crystallizes from acetone/ethanol. M. p. (HCl, decomp.): 240 °C.
¹H NMR (CDCl₃, δ, 400 MHz): 1.56 (br, s, 1H), 1.71 - 1.81 (m, 5H), 1.99 (dd, *J* = 12.6, 10.4 Hz, 1H), 2.14 (dd, *J* = 13.0, 10.1 Hz, 1H), 2.24 (ddd, *J* = 12.8, 8.8, 1.8 Hz, 1H), 2.57 (ddd, *J* = 12.1, 9.5, 2.2 Hz, 1H) 2.66 - 2.84 (m, 4H), 2.98 (dd, *J* = 12.5, 4.4 Hz, 1H), 3.05 (dd, *J* = 12.3,1.7 Hz, 1H), 3.21 (s, 2H), 3.76 (ddd, *J* = 12.6, 9.7, 2.9 Hz, 1H), 3.89 (br, t, *J* = 9.5 Hz, 1H), 7.08 (ddd, *J* = 8.8, 7.7, 1.5 Hz, 1H), 7.15 (ddd, *J* = 8.8, 7.7, 1.5 Hz, 1H), 7.22 -7.30 (m, 3H), 7.38 (dd, *J* = 8.1, 1.1 Hz, 1H), 7.77 (dd, *J* = 8.1,1.1 Hz, 1H), 7.92 (dd, *J* = 7.7,1.8 Hz, 1H) ppm.

### Example 14:

### (6RS,9RS)-(±)-6,9-Bis(2-chlorophenyl)-1-piperidino-3-azabicyclo[3.2.2]nonane

### (6RS,7RS)-(±)-6,7-Bis(2-chlorophenyl)-4-piperidinobicyclo[2.2.2]octan-2-one x HSCN

Piperidinium thiocyanate (24.5 g, 0.17 mol) and 4-(2-chlorophenyl)but-3-en-2-one (61.4 g, (0.34 mol) give after reaction and workup crystals (20.2 g, 24%).

### (7RS,8RS)-(±)-7,8-Bis(2-chlorophenyl)-5-piperidino-3-azabicyclo[3.2.2]nonan-2-one

The hydrothiocyanate of (6RS,7RS)-(±)-6,7-bis(2-chlorophenyl)-4-piperidinobicyclo[2.2.2]octan-2-one (4 g, 8.2 mmol) gives with NaN₃ (774 mg, 11.9 mmol) in concentrated sulfuric acid (25 ml) a resin (3.41 g, 94%).

### (6RS,9RS)-(±)-6,9-Bis(2-chlorophenyl)-1-piperidino-3-azabicyclo[3.2.2]nonane

The (7RS,8RS)-(±)-7,8-bis(2-chlorophenyl)-5-piperidino-3-azabicyclo[3.2.2]nonan-2-one (3.3 g, 7.5 mmol) gives with LiAlH₄ (1.42 g, 37 mmol) in dry ether (125 ml) a colourless resin (1.26 g, 39%). The hydrochloride crystallizes from acetone / ethanol. M.p. (HCl, decomp.): 297 °C.
¹H NMR (CDCl₃, δ, 400 MHz): 1.43 - 1.48 (m, 2H), 1.54 - 1.66 (m, 4H), 1.71 (br, s, 1H), 1.92 (br, t, *J* = 11.2 Hz, 1H), 2.04 (br, t, *J* = 11.6 Hz, 1H), 2.25 (ddd, *J* =12.8, 8.8, 1.8 Hz, 1H), 2.55 - 2.69 (m, 5H), 2.98 (dd, *J* = 12.3, 4.6 Hz, 1H), 3.05 (d, *J* = 12.1 Hz, 1H), 3.14 (d, *J* = 13.2 Hz, 1H), 3.20 (d, *J* = 13.2 Hz, 1H), 3.76 (ddd, *J* = 12.6, 9.7, 3.1 Hz, 1H), 3.84 (br, t, *J* = 9.5 Hz, 1H), 7.08 (ddd, *J* = 8.4, 7.3, 1.5 Hz, 1H), 7.15 (ddd, *J* = 8.8, 7.5, 1.5 Hz, 1H), 7.22 -7.30 (m, 3H), 7.37 (dd, *J* = 7.7, 1.1 Hz, 1H), 7.79 (dd, *J* = 7.7, 1.1 Hz, 1H), 7.93 (dd, *J* = 7.7, 1.1 Hz, 1H) ppm.

### Example 15:

### (6SR,9RS)-(±)-1-Dimethylamino-6,9-diphenyl-3-azabicyclo[3.2.2]nonane

### (6RS,7RS)-(±)-4-Dimethylamino-6,7-diphenylbicyclo[2.2.2]octan-2-one

The hydrothiocyanate of (6RS,7RS)-(±)-4-dimethylamino-6,7-diphenylbicyclo[2.2.2]octan-2-one (10 g, 26.4 mmol) is suspended in 2M NaOH (250 ml) and stirred for one hour. Subsequently, the suspension is extracted with ether till no solid remains between the two phases. The combined organic phases are washed 3 times with water, dried (Na₂SO₄), filtered and the solvent is evaporated in vacuo giving the base (8 g, 95 %) as a yellowish resin.

### (6SR,7RS)-(±)-4-Dimethylamino-6,7-diphenylbicyclo[2.2.2]octan-2-one

Method A, with tert. butanol as solvent:
   The (6RS,7RS)-(±)-4-dimethylamino-6,7-diphenylbicyclo[2.2.2]octan-2-one (3.5 g, 11 mmol) is dissolved in 32 g of dry tert. butanol and potassium tert. butylate (4.9 g, 44 mmol) is added. The mixture is refluxed over night at 145°C and then cooled to room temperature. Brine is added and the resulting solution is extracted 4 times with ether, the combined organic layers are washed with water, dried over sodium sulfate, filtered and the solvent is evaporated. The residue contains 35% of the isomerization product and 65% of starting material (¹H-NMR). It is dissolved in the minimum amount of ethanol, treated with 10 ml of concentrated HCl and is evaporated. This process is repeated several times. Small amounts of the hydrochloride of (6SR,7RS)-(±)-4-dimethylamino-6,7-diphenylbicyclo[2.2.2]octan-2-one are isolated by repeated crystallization from ethyl acetate / ethanol. M.p.(HCl): 261°C.
Method B: without solvent:
   The (6RS,7RS)-(±)-4-dimethylamino-6,7-diphenylbicyclo[2.2.2]octan-2-one (4.8 g, 15 mmol) is mixed with potassium tert. butylate (6.5 g, 58 mmol) and is heated to 200°C for 3 h. After cooling to room temperature, the dark red solid residue is dissolved in the minimum amount of EtOH and 500 ml of brine are added. The mixture is extracted 4 times with ether, the organic phases are combined, washed with water and dried over sodium sulfate. After filtration from the solid, the solvent is evaporated in vacuo. The residue contains 80% of the isomerization product and 20% of starting material. For further purification, the residue is dissolved in ethanol and concentrated aqueous HCl solution (10 ml) is added and the solvents are evaporated. This procedure is repeated once. The residue is dissolved in a mixture of ethanol / ethyl acetate and the hydrochloride of (6SR,7RS)-(±)-4-dimethylamino-6,7-diphenylbicyclo[2.2.2]octan-2-one (2.97 g, 56%) crystallizes over night. The base is set free by treatment of the hydrochloride with 2N NaOH and repeated extraxtion with ether. The combined organic phases are washed with water, dried (Na₂SO₄), filtered and the solvent is evaporated in vacuo. The resinous residue is dried under reduced pressure. M.p.(HCl): 261°C.

### (7SR,8RS)-(±)-5-Dimethylamino-7,8-diphenyl-3-azabicyclo[3.2.2]nonan-2-one

The hydrochloride of (6SR,7RS)-(±)-4-dimethylamino-6,7-diphenylbicyclo[2.2.2]octan-2-one reacts with NaN₃ dissolved in concentrated sulfuric acid to a yellow resin pure enough for further reactions.

### (6SR,9RS)-(±)-1-Dimethylamino-6,9-diphenyl-3-azabicyclo[3.2.2]nonane

The (7SR,8RS)-(±)-5-dimethylamino-7,8-diphenyl-3-azabicyclo[3.2.2]nonan-2-one gives with LiAlH₄ in dry ether a resin. The hydrochloride is prepared by repeated treatment with 2M ethereal HCl, evaporation of the solvent and crystallization.

### Example 16:

### (6SR,9RS)-(±)-1-Morpholino-6,9-diphenyl-3-azabicyclo[3.2.2]nonane

### (6SR,7RS)-(±)-4-Morpholino-6,7-diphenylbicyclo[2.2.2]octan-2-one

Method A: with tert. butanol as solvent:
   The (6RS,7RS)-(±)-4-morpholino-6,7-diphenylbicyclo[2.2.2]octan-2-one (14.6 g, 40 mmol) gives with potassium tert. butylate (7 g, 57 mmol) in dry tert. butanol (128 g) a resin which is purified by column chromatography over silica gel using benzene : ethyl acetate = 1 : 1 as eluent.
Method B: without solvent:
   The (6RS,7RS)-(±)-4-morpholino-6,7-diphenylbicyclo[2.2.2]octan-2-one (7.9 g, 22 mmol) gives with potassium tert. butylate (2.67 g, 22 mmol) after work-up 2.05 g (23%) hydrochloride of (6SR,7RS)-(±)-4-morpholino-6,7-diphenylbicyclo[2.2.2]octan-2-one as white solid. M.p.(HCl, decomp.): 274-278°C.

### (7SR,8RS)-(±)-5-Morpholino-7,8-diphenyl-3-azabicyclo[3.2.2]nonan-2-one

The hydrochloride of (6SR,7RS)-(±)-4-Morpholino-6,7-diphenylbicyclo[2.2.2]octan-2-one gives with NaN₃ in concentrated sulfuric acid a yellow resin pure enough for further reactions.

### (6SR,9RS)-(±)-1-Morpholino-6,9-diphenyl-3-azabicyclo[3.2.2]nonane

The (7SR,8RS)-(±)-5-morpholino-7,8-diphenyl-3-azabicyclo[3.2.2]nonan-2-one gives with LiAlH₄ in dry ether a resinous residue. The hydrochloride is prepared by repeated treatment with 2M ethereal HCl, evaporation of the solvent and crystallization.

### Example 17:

### (6SR,9RS)-(±)-6,9-Diphenyl-1-prrolidino-3-azabicyclo[3.2.2]nonane

### (6SR,7RS)-(±)-6,7-Diphenyl-4-pyrrolidinobicyclo[2.2.2]octan-2-one

The (6RS,7RS)-(±)-6,7-diphenyl-4-pyrrolidinobicyclo[2.2.2]octan-2-one (6.9 g, 20 mmol) gives with potassium tert. butylate (2.4 g, 20 mmol) after work-up 2.8 g (37%) of the hydrochloride of (6SR,7RS)-(±)-6,7-diphenyl-4-pyrrolidinobicyclo[2.2.2]octan-2-one. M.p.(HCl. decomp.): 275-278°C.

### (7SR,8RS)-(±)-7,8-Diphenyl-5-pyrrolidino-3-azabicyclo[3.2.2]nonan-2-one

The hydrochloride of (6SR,7RS)-(±)-6,7-diphenyl-4-pyrrolidinobicyclo[2.2.2]octan-2-one gives with NaN₃ in concentrated sulfuric acid a yellow resin pure enough for further reactions.

### (6SR,9RS)-(+)-6,9-Diphenyl-I-pyrrohdina-3-azabicyclo[3.2.2]nonane

The (7SR,8RS)-(±)-7,8-diphenyl-5-pyrrolidino-3-azabicyclo[3.2.2]nonan-2-one gives with LiAlH₄ in dry ether a resin. The hydrochloride is prepared by repeated treatment of solutions of the base with 2M ethereal HCl, evaporation of the solvent and crystallization.

### Example 18:

### (6SR,9RS)-(±)-6,9-Diphenyl-1-piperidino-3-azabicyclo[3.2.2]nonane

### (6SR,7RS)-(±)-6,7-Diphenyl-4-piperidinobicyclo[2.2.2]octan-2-one

The (6RS,7RS)-(±)-6,7-diphenyl-4-piperidinobicyclo[2.2.2]octan-2-one (7.67 g, 21 mmol) gives with potassium tert. butylate (2.6 g, 21 mmol) after work-up 2.9 g (35%) hydrochloride of (6SR,7RS)-(±)-6,7-diphenyl-4-piperidinobicyclo[2.2.2]octan-2-one. M.p.(HCl. decomp.): 275-285°C;

### (7SR,8RS)-(±)-7,8-Diphenyl-5-piperidino-3-azabicyclo[3.2.2]nonan-2-one

The hydrochloride of (6SR,7RS)-(±)-6,7-diphenyl-4-piperidinobicyclo[2.2.2]octan-2-one gives with NaN₃ in concentrated sulfuric acid a yellow resin pure enough for further reactions.

### (6SR,9RS)-(±)-6,9-Diphenyl-1-piperidino-3-azabicyclo[3.2.2]nonane

The (7SR,8RS)-(±)-7,8-diphenyl-5-piperidino-3-azabicyclo[3.2.2]nonan-2-one gives with LiAlH₄ in dry ether a resin. The hydrochloride is prepared by repeated treatment with 2M ethereal HCl, evaporation of the solvent and crystallization.

### Example 19:

### (6RS,9RS)-(±)-6-(4-Chlorophenyl)-9-phenyl-1-pyrrolidino-3-azabicyclo[3.2.2]nonane

### 4-Acetyl-3-(4-chlorophenyl)-5-phenylcyclohexanone

1. Preparation of 2-trimethylsiloxy-4-phenyl-1,3-butadiene:
   [Ref.: Y. Taniguchi, J. Inanga, M. Yamaguchi, Use of 1,8-Diazabicyclo[5.4.0]undec-7-ene in Preparation of Trimethylsilyl Enol Ethers and Trimethylsilylacetylenes. Bull. Chem. Soc. Jpn. 54, 3229-3230, (1981)]
   Benzylidene acetone (1.074 g, 7.35 mmol) is dissolved in dry CH₂Cl₂ (7.5 ml) and 1,8-Diazabicyclo[5.4.0]undec-7-ene (1.34 g, 8.8 mmol) is added. The mixture is cooled on an ice bath and trimethylsilylchloride (0.88 g, 8.1 mmol) is added under an atmosphere of Ar. After that, the mixture is heated up to 50°C on an oil bath and stirred for 30min at this temperature. After addition of 30 ml of pentane, the solution is washed 4 times with 1 % aqueous HCl and 3times with a saturated aqueous solution of NaHCO₃. The organic phase is dried (Na₂SO₄), filtered and the solvent evaporated giving 2-trimethylsiloxy-4-phenyl-1,3-butadiene (1.22 g, 76%) as a yellow liquid.
2. Preparation of 4-Acetyl-3-(4-chlorophenyl)-5-phenyl-cyclohexanone:
   In a two-necked dried round bottomed flask 4-(4-chlorophenyl)-but-3-en-2-one (2.3 g, 12.7 mmol) is dissolved in dry CH₂Cl₂ (80 ml) and triphenylcarbeniumpentachlorostannate (600 mg, 1.11 mmol) is added. The flask is filled with Ar and cooled to -78°C (solid CO₂, 2-propanol). 2-Trimethylsiloxy-4-phenyl-1,3-butadiene (3.6 g, 16.5 mmol) is added using a syringe which is washed with dry CH₂Cl₂ (10 ml). This solution is added, too. After 3h stirring at -78°C, a saturated aqueous solution of NaHCO₃ is added and the mixture is diluted with CH₂Cl₂ and filled in a separatory funnel. The organic phase is separated. The aqueous phase is extracted 3 times with CH₂Cl₂. The organic phases are combined and the solvent is evaporated in vacuo. The residue is treated with a mixture of THF (150 ml) and
   2M HCl (30 ml). It is filled in a separatory funnel and extracted 4 times with CH₂Cl₂. The combined organic layers are washed with water, dried (Na₂SO₄), filtered and the solvent evaporated giving 4.3 g residue which is purified using Kugelrohr distillation apparatus giving a yellow resin (2 g, 48%).
   ¹H NMR (CDCl₃, δ, 400 MHz): 1.66 (s, 3H), 2.66 - 2.71 (m, 2H), 3.00 (dd, *J* = 15.7, 6.0 Hz, 1H), 3.18 (dd, *J* = 15.0, 10.3 Hz, 1H), 3.40 (br, t, *J* = 5.9 Hz, 1H), 3.54 (ddd, *J* = 15.0, 10.3, 5.1 Hz, 1H), 3.60 (m,1H), 7.02 - 7.31 (m, 9H) ppm.

### (6RS,7RS)-(±)-6-(4-Chlorophenyl)-7-phenyl-4-pyrrolidinobicyclo[2.2.2]octan-2-one

4-Acetyl-3-(4-chlorophenyl)-5-phenylcyclohexanone (900 mg, 2.75 mmol), pyrrolidine (391 mg, 5.5 mmol), and concentrated HCl (0.22ml, 2.42mmol) are dissolved in 2-propanol (40 ml). The mixture is refluxed for two days at a water separator equipped with molecular sieves (3Å) at 130°C at an oil bath. Then the solvent is evaporated in vacuo giving a resin which is dissolved in CH₂Cl₂ and shaken 2 times with 2M NaOH and washed 3 times with water. The organic phase is dried (Na₂SO₄), filtered and the solvent evaporated giving a residue which is purified using column chromatography over silica gel using CH₂Cl₂ : CH₃OH = 10:1 as eluent. The fractions which contain the product are evaporated giving a resin which is purified by column chromatography over basic AhO₃ using CH₂Cl₂ as eluent giving a resin (500 mg, 48%). The hydrochloride is prepared by repeated treatment of a solution of the base in CH₂Cl₂ with 2M ethereal HCl, evaporation of the solvent and repeated crystallization from ethanol giving colourless plates. M.p. (HCl): 263°C.
¹H NMR (CDCl₃, δ, 400 MHz): 1.78 (ddd, *J* = 13.6, 10.6, 2.9 Hz, 1H), 1.82 (br, s, 4H), 2.11 (ddd, *J* = 13.6, 8.1, 2.4 Hz, 1H), 2.33 (ddd, *J* =13.2, 10.6, 2.6 Hz, 1H) 2.37 - 2.49 (m, 2H), 2.58 - 2.64 (m, 2H), 2.70 - 2.82 (m, 4H), 3.29 (br, t, *J* = 9.2 Hz, 1H), 3.34 (br, t, *J* = 9.5 Hz, 1H), 7.04 - 7.36 (m, 9H) ppm.

### (7RS,8RS)-(±)-7-(4-Chlorophenyl)-8-phenyl-5-pyrrolidino-3-azabicyclo[3.2.2]nonan-2-one

The hydrochloride of (6RS,7RS)-(±)-6-(4-chlorophenyl)-7-phenyl-4-pyrrolidinobicyclo[2.2.2]octan-2-one gives with NaN₃ in concentrated sulfuric acid a yellow resin pure enough for further reactions.

### (6RS,9RS)-(±)-6-(4-chlorophenyl)-9-phenyl-1-pyrrolidino-3-azabicyclo[3.2.2]nonane

The (7RS,8RS)-(±)-7-(4-chlorophenyl)-8-phenyl-5-pyrrolidino-3-azabicyclo[3.2.2]nonan-2-one gives with LiAll-L in dry ether a resinous residue. The hydrochloride is prepared by repeated treatment of solutions of the bases with 2M ethereal HCl, evaporation of the solvent and crystallization.

### Example 20:

### (6RS,9RS)-(±)-6-(4-Bromophehyl)-9-phenyl-1-pyrrolidino-3-azabicyclo[3.2.2]nonane

### 4-Acetyl-3-(4-bromophenyl)-5-phenylcyclohexanone

4-(4-Bromophenyl)but-3-en-2-one (2.25 g, 10.0 mmol) gives with 2-trimethylsiloxy-4-phenyl-1,3-butadiene (2.8 g, 12.8 mmol) in dry CH₂Cl₂ (60 ml) in the presence of triphenylcarbeniumpentachlorostannate (467 mg, 0.87 mmol) a resin (2.78 g, 75%).
¹H NMR (CDCl₃, δ, 400 MHz): 1.66 (s, 3H), 2.66 - 2.71 (m, 2H), 3.00 (dd, *J* = 15.9, 5.7 Hz, 1H), 3.17 (dd, *J* = 15.4, 9.9 Hz, 1H), 3.40 (br, t, *J* = 5.9 Hz, 1H), 3.53 - 3.60 (m, 2H), 7.02 - 7.47 (m, 9H) ppm.

### (6RS,7RS)-(±)-6-(4-Bromophenyl)-7-phenyl-4-pyrrolidinobicyclo[2.2.2]octan-2-one

4-Acetyl-3-(4-bromophenyl)-5-phenylcyclohexanone, pyrrolidine, and concentrated HCl dissolved in 2-propanol gives a resin. The hydrochloride is prepared by repeated treatment of a solution of the base in CH₂Cl₂ with 2M ethereal HCl, evaporation of the solvent and repeated crystallization from acetone/ethanol giving colourless plates. M.p. (HCl): 257°C.
¹H NMR (CDCl₃, δ, 400 MHz): 1.78 (ddd, *J* = 13.2, 8.4, 2.9 Hz, 1H), 1.81 (br, s, 4H), 2.11 (ddd, *J* = 12.8, 8.1, 2.6 Hz, 1H), 2.33 (ddd, *J* = 13.2, 10.6, 2.6 Hz, 1H), 2.41 - 2.49 (m, 2H), 2.58 -2.65 (m, 2H), 2.70 - 2.82 (m, 4H), 3.26 - 3.40 (m, 2H), 7.04 - 7.50 (m, 9H) ppm.

### (7RS,8RS)-(±)-7-(4-Bromophenyl)-8-phenyl-5-pyrrolidino-3-azabicyclo[3.2.2]nonan-2-one

The hydrochloride of (6RS,7RS)-(±)-6-(4-bromophenyl)-7-phenyl-4-pyrrolidinobicyclo[2.2.2]octan-2-one gives with NaN₃ in concentrated sulfuric acid a yellow resin pure enough for further reactions.

### (6RS,9RS)-(±)-6-(4-Bromophenyl)-9-phenyl-1-pyrrolidino-3-azabicyclo[3.2.2]nonane

The (7RS,8RS)-(±)-7-(4-bromophenyl)-8-phenyl-5-pyrrolidino-3-azabicyclo[3.2.2]nonan-2-one gives with LiAlH₄ in dry ether a resin. The hydrochloride is prepared by repeated treatment of solutions of the bases with 2M ethereal HCl, evaporation of the solvent and crystallization.

### Example 21:

### (6RS,9RS)-(±)-6-(4-Aminophenyl)-9-phenyl-1-pyrrolidino-3-azabicyclo[3.2.2]nonane

### 4-Acetyl-3-(4-nitrophenyl)-5-phenylcyclohexanone

4-(4-Nitrophenyl)but-3-en-2-one (2.3 g, 12 mmol) and 2-trimethylsiloxy-4-phenyl-1,3-butadiene (2.9 g, 13.5 mmol) give in dry CH₂Cl₂ (65 ml) in the presence of triphenylcarbeniumpentachlorostannate (490 mg, 0.91 mmol) a residue which is purified by column chromatography using cyclohexane : ethylacetate = 6:1 as eluent giving the pure product as a resin (680 mg,17%).
¹H NMR (CDCl₃, δ, 400 MHz): 1.74 (s, 3H), 2.70 - 2.78 (m, 2H), 3.03 (dd, *J* = 15.8, 5.5 Hz, 1H), 3.17 (dd, *J* = 15.6, 9.3 Hz, 1H), 3.49 (dd, *J* = 7.1, 5.3 Hz, 1H), 3.59 - 3.64 (m, 1H), 3.72 - 3.77 (m, 1H), 7.02 (d, *J* = 7.0 Hz, 2H), 7.25 - 7.34 (m, 3H), 7.41 (d, *J* = 8.8 Hz, 2H), 8.20 (d, *J* = 8.4 Hz, 2H) ppm.

### (6RS,7RS)-(±)-6-(4-Nitrophenyl)-7-phenyl-4-pyrrolidinobicyclo[2.2.2]octan-2-one

4-Acetyl-3-(4-nitrophenyl)-5-phenylcyclohexanone (1.42 g, 4.2 mmol), pyrrolidine (0.6 g, 8.4 mmol) and concentrated HCl (0.34 ml, 3.74 mmol) give in 2-propanol (60 ml) a resin (0.52 g, 38%). The hydrochloride is prepared by repeated treatment with 2M ethereal HCl, evaporation of the solvent and repeated crystallization from ethanol giving colourless plates. M.p. (HCl): 233°C.
¹H NMR (CDCl₃, δ, 400 MHz): 1.78 -1.86 (m, 5H), 2.17 (ddd, *J* = 12.8, 8.8, 2.2 Hz, 1H), 2.38 - 2.47 (m, 2H), 2.51 (dd, *J* =18.5, 2.4 Hz, 1H), 2.65 (dd, *J* = 18.5, 3.1 Hz, 1H), 2.69 (s,1H), 2.70 - 2.82 (m, 4H), 3.25 (t, *J* = 9.5 Hz, 1H), 3.47 (t, *J* = 9.3 Hz, 1H), 7.05 (d, *J* = 7.0 Hz, 2H), 7.17 - 7.28 (m, 3H), 7.53 (d, *J* = 8.8 Hz, 2H), 8.25 (d, *J* = 8.8 Hz, 2H) ppm.

### (7RS,8RS)-(±)-7-(4-Nitrophenyl)-8-phenyl-5-pyrrolidino-3-azabicyclo[3.2.2]nonan-2-one

The hydrochloride of (6RS,7RS)-(±)-6-(4-nitrophenyl)-7-phenyl-4-pyrrolidinobicyclo[2.2.2]octan-2-one gives with NaN₃ in concentrated sulfuric acid a yellow resin pure enough for further reactions.

### (7RS,8RS)-(±)-7-(4-Aminophenyl)-8-phenyl-5-pyrrolidino-3-azabicyclo[3.2.2]nonan-2-one

The (7RS,8RS)-(±)-7-(4-nitrophenyl)-8-phenyl-5-pyrrolidino-3-azabicyclo[3.2.2]nonan-2-one (570 mg, 1.4 mmol) is dissolved in ethanol (100 ml) and SnCl₂.2H₂O (1.43 g, 6.34 mmol) is added. The mixture is stirred over night and then another portion of SnCl₂.2H₂O (794 mg, 3.52 mmol) is added. The mixture is stirred over night, then 2 M NaOH is added and the mixture is extracted 5 times with CH₂Cl₂, the combined organic phases are washed with water, dried (Na₂SO₄), filtered and the solvent evaporated giving a residue (500 mg, 89%).

### (6RS,9RS)-(±)-6-(4-Aminophenyl)-9-phenyl-1-pyrrolidino-3-azabicyclo[3.2.2]nonane

To (7RS,8RS)-(±)-7-(4-aminophenyl)-8-phenyl-5-pyrrolidino-3-azabicyclo[3.2.2]nonan-2-one (63 mg, 0.17 mmol) Red-Al^{®} solution (3.4 mM in toluene, 0.7 ml) is added and the mixture is subjected to microwave irradiation at 100°C for 15 min. The reaction is quenched cautiously with 2 M NaOH and extracted 4 times with toluene. The combined organic phases are washed with water, dried (Na₂SO₄ filtered and the solvent evaporated giving a residue which is purified by column chromatography over basic aluminium oxide using ethyl acetate/cyclohexane/methanol = 3/1/1 as eluent giving a resin (35 mg, 56%).
¹H NMR (CDCl₃, δ, 400 MHz): 1.75 (br, s, 4H), 1.94 (br, s, 1H), 1.96 -2.08 (m, 2H), 2.25 (ddd, *J* = 12.8, 9.2,1.8 Hz, 1H), 2.46 (ddd, *J* = 12.1,10.3,1.8 Hz, 1H), 2.64 - 2.82 (m, 4H), 2.86 (d, *J* = 12.8 Hz, 1H), 3.09 (dd, *J* = 12.8, 4.4 Hz, 1H), 3.13 (s, 2H), 3.26 (t, *J* = 9.5 Hz, 1H), ), 3.33 (ddd, *J* = 9.7, 7.0, 2.7 Hz, 1H), 3.59 (br, s, 2H), 6.67 (d, *J* = 8.1 Hz, 2H), 7.16 -7.38 (m, 7H) ppm.

### Example 22:

### (6RS,9RS)-(±)-1-(N-Methyl-N-(pyridin-3-ylmethyl)amino)-6,9-diphenyl-3-azabicyclo[3.2.2]nonane

### (5RS,8RS)-(±)-N-methyl-N-(3-oxo-5,8-diphenylbicyclo[2.2.2]octan-1-yl)formamide

The (6RS,7RS)-(±)-4-dimethylamino-6,7-diphenylbicyclo[2.2.2]octan-2-one (1.94 g, 6.1 mmol) is dissolved in acetone (75 ml). Then KMnO₄ (3.8 g, 24 mmol) is added under stirring and cooling in portions. The reaction mixture is stirred overnight at ambient temperature and the precipitated MnO₂ is filtered off. The MnO₂ is washed repeatedly with hot acetone and the washing solutions filtered and combined with the filtrate. After evaporation of the solvent in vacuo the residue is dissolved in ether and extracted five times with diluted HCl and two times with water. After drying over Na₂SO₄, the solution is filtered and the solvent evaporated in vacuo. The residue is purified by means of column chromatography over silica gel using CH₂Cl₂ : CH₃OH = 20 : 1 as eluens giving the product as a colourless resin 1.6 g (80%) which contains 73% E-isomer and 27% Z-isomer.
(E)-(5RS,8RS)-(±)-N-Methyl-N-(3-oxo-5,8-diphenylbicyclo[2.2.2]octan-1-yl)formamide: ¹H NMR (CDCl₃, δ, 400 MHz): 2.02 (ddd, *J* = 13.0, 7.7, 2.9 Hz, 1H), 2.39 - 2.53 (m, 2H), 2.60 - 2.78 (m, 2H), 2.78 (s, 1H) 2.86 (dd, *J* = 17.9, 3.7 Hz, 1H), 2.98 (s, 3H), 3.45 - 3.49 (m, 2H), 7.03 - 7.43 (m, 10H), 8.50 (s, 1H) ppm.
(Z)-(SRS,8RS)-(±)-N-Methyl-N-(3-oxo-5,8-diphenylbicyclo[2.2.2]octan-1-yl)formamide:¹H NMR (CDCl₃, δ, 400 MHz): 2.11 (ddd, *J* = 13.0, 7.7, 2.7 Hz, 1H), 2.57 - 2.73 (m, 2H), 2.71 (s, 1H), 2.75 - 2.80 (m, 2H), 3.00 (s, 3H), 3.28 (dd, *J* = 18.6, 3.6 Hz, 1H), 3.31 - 3.44 (m, 2H), 7.03-7.43 (m, 10H), 8.16 (s,1H) ppm.

### (6RS,7RS)-(±)-4-Methylamino-6,7-diphenylbicyclo[2.2.2]octan-2-one

The (5RS,8RS)-(±)-N-methyl-N-(3-oxo-5,8-diphenylbicyclo[2.2.2]octan-1-yl)formamide (2.88 g, 8.6 mmol) is suspended in diethyleneglycole (35 ml). Then KOH (2.67 g, 47.6 mmol) is added and the reaction mixture is heated to 140°C and stirred at this temperature for 150 min. After cooling, water is added and the mixture is extracted five times with CH₂Cl₂. The combined organic layers are washed with water, dried over Na₂SO₄, filtered and the solvent is evaporated in vacuo. The residue is purified by means of column chromatograpy over silica gel using CH₂Cl₂ : MeOH = 20: 1.5 as eluent giving the product as colourless resin (2.3 g, 90%). The hydrochloride is prepared by repeated treatment of a solution of the base with 2M ethereal HCl, evaporation of the solvent and crystallization from ethanol/ethyl acetate. M.p. (HCl):181°C.
¹H NMR (CDCl₃, δ*,* 400 MHz): 1.76 (ddd, *J* = 13.1, 7.8, 2.8 Hz, 1H), 2.08 (ddd, *J* = 13.0, 7.9, 2.6 Hz, 1H), 2.19 (ddd, *J* = 13.4, 10.8, 2.9 Hz, 1H), 2.27 (ddd, *J* = 13.1, 10.7, 3.2 Hz, 1H), 2.36 (dd, *J* = 18.5, 2.5 Hz, 1H), 2.44 (s, 3H), 2.54 (dd, J = 18.5, 3.2 Hz, 1H), 2.67 (s, 1H), 3.39 (t, *J* = 9.3 Hz, 2H), 7.03 - 7.40 (m, 10H) ppm.

### (7RS,8RS)-(±)-5-Methylamino-7,8-diphenyl-3-azabicyclo[3.2.2]nonan-2-one

The hydrochloride of (6RS,7RS)-(t)-4-methylamino-6,7-diphenylbicyclo[2.2.2]octan-2-one (1.12 g, 3.28 mmol) is dissolved in concentrated sulfuric acid (11 ml) under stirring and cooling on an ice bath. The solution is allowed to warm up to room temperature and NaN₃ (350 mg, 5.38 mmol) is added in portions. After the addition is completed, the solution is poored on ice and alkalized with 2M NaOH and extracted 5 times with CH₂Cl₂. The combined organic phases are washed 3 times with water, dried over sodium sulfate, filtered and the solvent is evaporated in vacuo giving the product as yellow resin (980 mg, 93%).
¹H NMR (CDCl₃, δ, 400 MHz): 1.99 (dd, *J* = 13.0, 8.6 Hz, 1H), 2.07 - 2.17 (m, 3H), 2.35 (s, 3H), 2.86 (br, s, 1H), 3.19 (dd, *J* = 12.5, 2.7 Hz, 1H), 3.31 (d, *J* = 12.5 Hz, 1H), 3.42 (ddd, *J* = 12.1, 9.7, 2.4 Hz, 1H), 3.47 (dd, *J*= 10.8, 8.6 Hz, 1H), 6.96 (br, s, 1H), 7.17 - 7.38 (m, 10H) ppm.

### (6RS,9RS)-(±)-1-Methylamino-6,9-diphenyl-3-azabicyclo[3.2.2]nonane

The (7RS,8RS)-(±)-5-methylamino-7,8-diphenyl-3-azabicyclo[3.2.2]nonan-2-one (360 mg, 1.12 mmol) is suspended in dry ether (16 ml) with stirring and cooling. Powdered LiAlH₄ (180 mg, 4.72 mmol) is added in portions. Then the suspension is refluxed over night, cooled to room temperature and quenched with water cautiously under stirring and cooling on an ice bath. 2N NaOH is added and the mixture is extracted five times with ether, the combined organic phases are washed twice with water, dried (Na₂SO₄ filtered and the solvent evaporated giving a resinous residue (300 mg, 87%). The hydrochloride is prepared by repeated treatment of a solution of the base with 2M ethereal HCl, evaporation of the solvent and crystallization from ethanol/ethyl acetate. M.p. (HCl): 216-218°C.
¹H NMR (CDCl₃, δ, 400 MHz): 1.89 - 1.96 (m, 2H), 2.06 (d, *J* = 2.9 Hz, 1H), 2.11 (ddd, *J* = 12.6, 10.3, 2.4 Hz, 1H), 2.39 (s, 3H), 2.44 (ddd, *J* = 12.8, 8.8, 2.2 Hz, 1H), 2.87 (dd, *J* = 12.8, 2.1 Hz, 1H), 2.97 (d, *J* = 13.6 Hz, 1H), 3.08 - 3.12 (m, 2H), 3.35 - 3.43 (m, 2H), 7.17 - 7.40 (m, 10H) ppm.

### (6RS,9RS)-(±)-1-(N-Methyl-N-(pyridin-3-ylmethyl)amino)-6,9-diphenyl-3-azabicyclo[3.2.2]nonane

The (6RS,9RS)-(±)-1-methylamino-6,9-diphenyl-3-azabicyclo[3.2.2]nonane (200 mg, 0.62 mmol) is dissolved in dry CH₂Cl₂ (5 ml) and nicotinoyl chloride hydrochloride (167 mg, 0.94 mmol) is added with stirring and cooling. Then a mixture of triethylamine (95 mg, 0.94 mmol) and dry CH₂Cl₂ (5 ml) is added dropwise. The mixture is allowed to warm up to room temperature and is stirred for 3 days under an Ar-atmosphere. The mixture is diluted with CH₂Cl₂ and water is added. It is shaken twice thoroughly with 2N NaOH and washed with water. After drying (Na₂SO₄), the organic phase is filtered and the solvent evaporated giving a resinous residue (250 mg, 0.59 mmol) of the amide which is dried by repeated co-distillation with dry benzene. It is suspended in dry ether (10 ml). LiAlH₄ (170 mg, 4.45 mmol) is added in portions with stirring and cooling. The mixture is refluxed over night and then quenched cautiously by addition of water and 2N NaOH with stirring and cooling. The mixture is extracted 5 times with ether and the organic layer is washed twice with water, dried (Na₂SO₄) and filtered. The solvent is evaporated in vacuo and the residue purified by means of column chromatography with CH₂Cl₂ : CH₃OH = 1 : 1 as eluent giving the product as a colourless resin. The hydrochloride is prepared by repeated treatment with 2 M ethereal HCl. It is recrystallized from a mixture of ethanol/ethyl acetate giving white crystals (65 mg, 21%). M.p.(HCl): 241°C.
¹H NMR (CDCl₃, δ, 400 MHz): 1.98 - 2.10 (m, 3H), 2.16 (s, 3H), 2.36 (ddd, *J* = 12.5, 8.8, 3.7 Hz, 1H), 2.58 (ddd, *J* = 11.4, 9.9, 1.5 Hz, 1H), 2.93 (d, *J* = 12.8 Hz, 1H), 3.18 (dd, *J* = 13.2, 4.4 Hz, 1H), 3.20 (s, 2H), 3.37 - 3.46 (m, 3H), 3.84 (d, *J* = 13.9 Hz, 1H), 7.19 - 7.45 (m, 11H), 7.69 (d, *J* = 7.7 Hz, 1H), 8.48 (d, *J* = 3.3 Hz, 1H), 8.54 (s,1H) ppm.

### Example 23:

### (6RS,9RS)-(±)-1-(N-(2-Diethylaminoethyl)-N-methylanylamino)-6,9-diphenyl-3 azabicyclo[3.2.2]nonane

### (6RS,9RS)-(±)-2-Chloro-N-methyl-N-(4-oxo-6,9-diphenyl-3-azabicyclo[3.2.2]non-1-yl)acetamide

The (7RS,8RS)-(±)-5-methylamino-7,8-diphenyl-3-azabicyclo[3.2.2]nonan-2-one (1.4 g, 4.4 mmol) is dissolved in dry CH₂Cl₂ (60 ml) and cooled with stirring under an atmosphere of Ar on an ice bath. Then, chloroacetyl chloride (0.75 g, 6.6 mmol), dissolved in dry CH₂Cl₂ (30 ml) and triethylamine (0.67 g, 6.6 mmol) dissolved in dry CH₂Cl₂ (30 ml) are added to the cooled mixture. The ice bath is removed and the mixture is stirred over night at room temperature. 1M NaOH and CH₂Cl₂ are added and the mixture is shaken. The organic phase is washed 3 times with water, dried (Na₂SO₄), filtered and the solvent evaporated in vacuo giving a resin (1.7 g, 97%).

### (6RS,9RS)-(±)-2-Diethylamino-N-methyl-N-(4-oxo-6,9-diphenyl-3-azabicyclo[3.2.2]non-1-yl)acetamide

To the (6RS,9RS)-(±)-2-chloro-N-methyl-N-(4-oxo-6,9-diphenyl-3-azabicyclo[3.2.2]non-1-yl)acetamide (1.7 g, 4.3 mmol) diethylamine (22.9 g, 0.31 mol) dissolved in ethanol (12 ml) and a catalytic amount of KI is added. The solution is stirred over night at room temperature under an atmosphere of Ar. Benzene is added to the solution and the solvents are evaporated. The residue is dissolved in CH₂Cl₂ and shaken with 1N NaOH. The organic phase is separated and washed with water until the water phase reacts neutral. Then the organic phase is dried (Na₂SO₄), filtered and the solvent evaporated in vacuo giving a resin which is purified by column chromatography over silica gel using CH₂Cl₂: CH₃OH = 10:3 yielding a colourless resin (486 mg, 26% ).
¹H NMR (CDCl₃, δ, 400 MHz): 1.02 (t, *J* = 7.0 Hz, 6H), 2.18 - 2.25 (m, 2H), 2.58 (q, *J* = 7.0 Hz, 4H), 2.72 (ddd, *J* = 12.3,8.1, 1.5 Hz, 1H), 2.87 (s, 1H), 3.10 (s, 3H), 3.19 - 3.27 (m, 3H), 3.43 - 3.51 (m, 2H), 3.71 (d, *J* = 12.5 Hz, 1H), 3.87 (ddd, *J* = 12.5, 5.1,1.5 Hz, 1H), 6.21 (br, d, *J* = 2.9 Hz, 1H), 7.18 - 7.39 (m, 10H) ppm.

### (6RS,9RS)-(±)-1-(N-(2-Diethylaminoethyl)-N-methylamino)-6,9-diphenyl-3-azabicyclo[3.2.2]nonane

The (6RS,9RS)-(±)-2-diethylamino-N-methyl-N-(4-oxo-6,9-diphenyl-3-azabicyclo[3.2.2]non-1-yl)acetamide (486 mg,1.12 mmol) is suspended in dry ether (19 ml) with stirring and cooling. Powdered LiAlH₄ (255 mg, 6.7 mmol) is added in portions. The suspension is refluxed two days, cooled to room temperature and quenched cautiously with water under stirring and cooling on an ice bath. 2N NaOH is added and the mixture is extracted with ether. The combined organic phases are washed twice with water, dried (Na₂SO₄), filtered and the solvent evaporated giving a resin (413 mg, 91 %).
¹H NMR (CDCl₃, δ, 400 MHz): 1.03 (t, *J* = 7.0 Hz, 6H), 1.91 - 2.00 (m, 3H), 2.23 (ddd, *J* = 12.5, 8.8,3.7 Hz, 1H), 2.30 (s, 3H), 2.35 - 2.41 (m, 1H), 2.46 - 2.57 (m, 7H), 2.75 - 2.82 (m, 1H), 2.88 (d, *J* = 12.8 Hz, 1H), 3.04 - 3.13 (m, 3H), 3.32 - 3.36 (m, 2H), 7.17 - 7.40 (m, 10H) ppm.

### Example 24:

### (6RS,9RS)-(±)-1-(N-(2-Dipropylaminoethyl)-N-methylamino)-6,9-diphenyl-3-azabicyclo[3.2.2]nonane

### (6RS,9RS)-(±)-2-Dipropylamino-N-methyl-N-(4-oxo-6,9-diphenyl-3-azabicyclo[3.2.2]non-1-yl)acetamide

(6RS,9RS)-(±)-2-Chloro-N-methyl-N-(4-oxo-6,9-diphenyl-3-azabicyclo[3.2.2]non-1-yl)acetamide (1.8 g, 4.5 mmol) gives with dipropylamine (33.1 g, 0.322 mmol) in ethanol (10 ml) in the presence of KI a resin which is purified by column chromatography over silica gel using CH₂Cl₂ : CH₃OH = 40:1 yielding a resin (506 mg, 24% ). ¹H NMR (CDCl₃, δ, 0400 MHz): 0.87 (t, *J* = 7.3 Hz, 6H), 1.45 (q, *J* = 7.3 Hz, 4H), 2.17 - 2.24 (m, 2H), 2.43 (br, t, *J* = 6.4 Hz, 4H), 2.71 (dd, *J* = 12.3, 8.2 Hz, 1H), 2.87 (s, 1H), 3.12 (s, 3H), 3.20 - 3.26 (m, 3H), 3.43 - 3.51 (m, 2H), 3.70 (d, *J* = 12.1 Hz, 1H), 3.89 (dd, *J* = 12.3, 4.9 Hz, 1H), 6.15 (br, s, 1H), 7.18 - 7.39 (m, 10H) ppm.

### (6RS,9RS)-(±)-1-(N-(2-Dipropylaminoethyl)-N-methylamino)-6,9-diphenyl-3-azabicyclo[3.2.2]nonane

The (6RS,9RS)-(±)-2-dipropylamino-N-methyl-N-(4-oxo-6,9-diphenyl-3-azabicyclo[3.2.2]non-1-yl)acetamide (506 mg,1.1 mmol) gives with LiAlH₄ (250 mg, 6.6 mmol) in dry ether (20 ml) a resin (293 mg, 62%).
¹H NMR (CDCl₃, δ, 400 MHz): 0.87 (t, *J* = 7.3 Hz, 6H), 1.47 (q, *J* = 7.3 Hz, 4H),1.90 - 2.00 (m, 3H), 2.22 (t, *J* = 12.1 Hz, 1H), 2.30 (s, 3H), 2.32 - 2.42 (m, 5H), 2.45 - 2.54 (m, 3H), 2.72 - 2.79 (m, 1H), 2.87 (d, *J* = 12.8 Hz, 1H), 3.04 - 3.14 (m, 3H), 3.34 (t, br, *J* = 9.5 Hz, 2H), 7.17 - 7.40 (m, 10H) ppm.

### Example 25:

### (6RS,9RS)-(±)-1-(N-Methyl-N-(2-pvrrohdinoethyl)amino)-6,9-diphenyl-3-azabicyclo[3.2.2]nonane

### (6RS,9RS)-(±)-N-Methyl-N-(4-oxo-6,9-diphenyl-3-azabicyclo[3.2.2]non-1-yl)-2-pyrrolidino-acetamide

(6RS,9RS)-(±)-2-Chloro-N-methyl-N-(4-oxo-6,9-diphenyl-3-azabicyclo[3.2.2]non-1-yl)acetamide (923 mg, 2.3 mmol) gives with pyrrolidine (6.0 g, 84 mmol) in the presence of KI a resin which is purified by column chromatography using CH₂Cl₂: CH₃OH = 20:1 yielding a colourless resin (310 mg, 31 %).
¹H NMR (CDCl₃, δ, 400 MHz): 1.79 (br, s, 4H), 2.21 (t, *J* = 11.2 Hz, 2H), 2.66 - 2.71 (m, 5H), 2.85 (s, 1H), 3.04 (s, 3H), 3.22 (t, *J* = 12.5 Hz, 1H), 3.37 - 3.50 (m, 4H), 3.65 - 3.70 (m, 1H), 3.89 (dd, *J* = 12.5, 4.0 Hz, 1H), 6.47 (s, 1H), 7.16 - 7.38 (m, 10H) ppm.

### (6RS,9RS)-(±)-1-(N-Methyl-N-(2-pyrrolidinoethyl)amino)-6,9-diphenyl-3-azabicyclo[3.2.2]nonane

The (6RS,9RS)-(±)-N-methyl-N-(4-oxo-6,9-diphenyl-3-azabicyclo[3.2.2]non-1-yl)-2-pyrrolidino-acetamide (260 mg, 0.6 mmol) gives with LiAlH₄ (137 mg, 3.6 mmol) in dry ether (10 ml) a resin (226 mg, 93%).
¹H NMR (CDCl₃, δ, 400 MHz): 1.74 - 1.82 (m, 4H), 1.91 - 2.00 (m, 3H), 2.24 (ddd, *J* = 12.8, 8.8, 2.2 Hz, 1H), 2.32 (s, 3H), 2.43 - 2.59 (m, 8H), 2.81 - 2.89 (m, 2H), 3.05 - 3.14 (m, 3H), 3.31 - 3.36 (m, 2H), 7.17 - 7.40 (m, 10H) ppm.

### Example 26:

### (6RS,9RS)-(±)-1-(N-Methyl-N-(2-(4-methylpiperazin-1-yl)ethyl)amino)-6,9-diphenyl-3-azabicyclo[3.2.2]nonane

### (6RS,9RS)-(±)-N-Methyl-2-(4-methylpiperazin-1-yl)-N-(4-oxo-6,9-diphenyl-3-azabicyclo[3.2.2]non-1-yl)acetamide

(6RS,9RS)-(±)-2-Chloro-N-methyl-N-(4-oxo-6,9-diphenyl-3-azabicyclo[3.2.2]non-1-yl)acetamide (0.8 g, 2.1 mmol) gives with N-methylpiperazine (7.4 g, 74.4 mmol) in the presence of KI a resin which is purified by column chromatography over aluminium oxide using CH₂Cl₂ : CH₃OH = 24 :1 yielding a resin (515 mg, 54%).
¹H NMR (CDCl₃, δ, 400 MHz): 2.17 - 2.25 (m, 2H), 2.30 (s, 3H), 2.42 - 2.62 (m, 8H), 2.70 (dd, J = 11.9, 8.6 Hz, 1H), 2.87 (s, 1H), 3.05 (s, 3H), 3.11 (d, *J* = 14.3 Hz, 1H), 3.17 (d, *J* = 14.3 Hz, 1H), 3.22 (t, *J* = 13.2 Hz, 1H), 3.43 - 3.51 (m, 2H), 3.70 (d, *J* = 12.1 Hz, 1H), 3.88 (dd, *J* = 12.5, 4.4 Hz, 1H), 6.22 (d, *J* = 4.4 Hz, 1H), 7.18 - 7.39 (m, 10H) ppm.

### (6RS,9RS)-(±)-1-(N-Methyl-N-(2-(4-methylpiperazin-1-yl)ethyl)amino)-6,9-diphenyl-3-azabicyclo[3.2.2]nonane

The (6RS,9RS)-(±)-N-methyl-2-(4-methylpiperazin-1-yl)-N-(4-oxo-6,9-diphenyl-3-azabicyclo[3.2.2]non-1-yl)acetamide (463 mg, 1 mmol) gives with LiAlH₄ (229 mg, 6 mmol) in dry ether (20 ml) a resin (372 mg, 86%).
¹H NMR (CDCl₃, *δ,* 400 MHz): 1.90 - 1.99 (m, 3H), 2.23 (ddd, *J* = 12.8, 9.2, 1.8 Hz, 1H), 2.28 (s, 3H), 2.30 (s, 3H), 2.36 - 2.62 (m, 12H), 2.80 - 2.89 (m, 2H), 3.04 - 3.14 (m, 3H), 3.31 - 3.35 (m, 2H), 7.17 - 7.41 (m, 10H) ppm.

### Example 27:

### (6RS,9RS)-(±)-1-(N-Methyl-N-(2-piperidinoethyl)amino)-6,9-diphenyl-3-azabicyclo[3.2.2]nonane

### (6RS,9RS)-(±)-N-Methyl-N-(4-oxo-6,9-diphenyl-3-azabicyclo[3.2.2]non-1-yl)-2-piperidino-acetamide

(6RS,9RS)-(±)-2-Chloro-N-methyl-N-(4-oxo-6,9-diphenyl-3-azabicyclo[3.2.2]non-1-yl)acetamide (0.8 g, 2.1 mmol) gives with piperidine (6.3 g, 74 mmol) in the presence of a catalytic amount of KI a resin which is purified by column chromatography over aluminium oxide using CH₂Cl₂ : CH₃OH = 34 :1 as eluent yielding a resin (426 mg, 46%).
¹H NMR (CDCl₃, δ, 400 MHz): 1.36 - 146 (m, 2H), 1.54 - 1.60 (m, 4H), 2.17 - 2.25 (m, 2H), 2.42 (s, 4H), 2.70 (dd, *J* = 11.7, 8.4 Hz, 1H), 2.87 (s, 1H), 3.08 (s, 3H), 3.10 - 3.11 (m, 2H), 3.23 (t, *J* = 12.8 Hz, 1H), 3.43 - 3.51 (m, 2H), 3.70 (d, *J* = 12.5 Hz, 1H), 3.88 (dd, *J*= 12.5, 4.0 Hz, 1H), 6.21 (d, *J* = 4.0 Hz, 1H), 7.17 - 7.39 (m, 10H) ppm.

### (6RS,9RS)-(±)-1-(N-Methyl-N-(2-piperidinoethyl)amino)-6,9-diphenyl-3-azabicyclo[3.2.2]nonane

The (6RS,9RS)-(±)-N-methyl-N-(4-oxo-6,9-diphenyl-3-azabicyclo[3.2.2]non-1-yl)-2-piperidino-acetamide gives with LiAlH₄ in dry ether a resin.

### Example 28:

### (6RS,9RS)-(±)-1-(N-(3-Aminopropyl)-N-methylaniino)-6,9-diphenyl-3-azabicyclo[3.2.2]nonane

### (6RS,9RS)-(±)-2-Cyano-N-methyl-N-(4-oxo-6,9-diphenyl-3-azabicyclo[3.2.2]non-1-yl)acetamide

2-Cyanoacetic acid (561 mg, 6.6 mmol) is stirred in dry diethyl ether under an argon atmosphere with cooling on an ice bath. Phoshorous pentachloride (1.4 g, 6.6 mmol) is added in portions within 10 min. The ice bath is removed and the mixture is stirred for an additional hour at room temperature. The solvent is removed in vacuo, 2 ml of benzene are added and removed in vacuo at 60°C. This procedure is repeated to remove POCl₃. (7RS,8RS)-(±)-5-Methylamino-7,8-diphenyl-3-azabicyclo[3.2.2]nonan-2-one (1.27 g, 4 mmol) is dissolved in dry CH₂Cl₂ (20 ml) and the 2-cyanoacetyl chloride, dissolved in dry CH₂Cl₂ (10 ml) is added under stirring and cooling on an ice bath. Triethylamine (600mg, 5.3 mmol) diluted with dry CH₂Cl₂ (30 ml) is added and a balloon, filled with Ar, is placed on the round bottomed flask to ensure Ar atmosphere. The mixture is stirred for 3 days at room temperature giving a yellow solution which is filled in a separatory funnel. 2M NaOH is added to alkalize the solution. This mixture is repeatedly extracted with ether, the combined organic phases are washed with brine, dried (Na₂SO₄), filtered and the solvent evaporated giving a white resin (903 mg, 59%).
¹H NMR (CDCl₃, δ, 400 MHz): 2.17 - 2.22 (m, 2H), 2.64 (dd, *J* = 12.2, 7.9 Hz, 1H), 2.85 (s, 1H), 2.93 (s, 3H), 3.12 (t, *J* = 12.6 Hz, 1H), 3.41 - 3.48 (m, 2H), 3.49 (s, 2H), 3.64 (d, *J* = 12.5 Hz, 1H), 3.75 (dd, *J* = 12.5, 5.1 Hz, 1H), 6.44 (d, *J* = 3.3 Hz, 1H), 7.17 - 7.36 (m, 10H) ppm.

### (6RS,9RS)-(±)-1-(N-(3-Aminopropyl)-N-methylamino)-6,9-diphenyl-3-azabicyclo[3.2.2]nonane

(6RS,9RS)-(±)-2-Cyano-N-methyl-N-(4-oxo-6,9-diphenyl-3-azabicyclo[3.2.2]non-1-yl)acetamide and Red-Al^{®} solution (3.4 mM in toluene) gives under microwave irradiation a resin.

### Example 29:

### (6RS,9RS)-(±)-3-(2-Aminoethyl)-6,9-diphenyl-1-pyrrolidino-3-azabicyclo[3.2.2]nonane

(6RS,9RS)-(±)-6,9-Diphenyl-1-pyrrolidino-3-azabicyclo[3.2.2]nonane (874 mg, 2.5 mmol) is dissolved in dry ethanol (20 ml) and cooled on an ice bath with stirring under an atmosphere of Ar. A solution of chloroacetamide (236 mg, 2.5 mmol) in dry ethanol (12 ml) is added. The mixture is refluxed for two days, allowed to cool to room temperature, diluted with water and alkalized with 2M NaOH. Then it is extracted 4 times with ether. The combined organic phases are washed with water, dried (Na₂SO₄) and filtered and the solvent evaporated giving a resinous residue which is purified using column chromatography and CH₂Cl₂: MeOH = 3 : 2 as eluent giving (6RS,9RS)-(±)-2-(6,9-diphenyl-1-pyrrolidino-3-azabicyclo[3.2.2]non-3-yl)acetamide (660 mg, 1.6 mmol) as a colourless resin which is suspended in dry ether (20 ml) under stirring and cooling on an ice bath. LiAlH₄ (300 mg, 7.9 mmol) is added in portions and the mixture is refluxed over night. The reaction is cautiously quenched with ice water. 2M NaOH is added and the mixture is extracted 5 times with CHCl₃, the combined organic phases are washed twice with water, dried (Na₂SO₄) and filtered and the solvent evaporated giving a colourless resin (625 mg, 63%). The hydrochloride is prepared by repeated treatment with 2M ethereal HCl and subsequent evaporation of the solvent. Then it is crystallized twice from ethanol/acetone giving a white powder. M.p. (HCl): 228°C.
¹H NMR (CDCl₃, δ, 400 MHz): 1.76 (br, s, 4H), 1.87 - 2.05 (m, 3H), 2.29 - 2.35 (m, 2H), 2.49 - 2.61 (m, 2H), 2.63 - 2.90 (m, 8H), 2.97 - 3.03 (m, 2H), 3.30 - 3.36 (m, 2H), 7.16 - 7.42 (m, 10H) ppm.

### Example 30:

### (6RS,9RS)-(±)-3-(2-Diethylaninoethyl)-1-dimethylamnino-6,9-dighenyl-3-azabicyclo[3.2.2]nonane

### (6RS,9RS)-(±)-2-Chloro-N-(1-dimethylamino-dno-6,9-diphenyl-3-azabicyclo[3.2.2]non-3-yl)acetamide

(6RS,9RS)-(±)-1-Dimethylamino-6,9-diphenyl-3-azabicyclo[3.2.2]nonane is dissolved in dry CH₂Cl₂ and cooled with stirring under an atmosphere of Ar on an ice bath. Then, chloroacetyl chloride dissolved in dry CH₂Cl₂ and triethylamine dissolved in dry CH₂Cl₂ are added to the cooled mixture. The mixture is stirred over night at room temperature. 1M NaOH and CH₂Cl₂ are added and the mixture is shaken. The organic phase is washed 3 times with water, dried (Na₂SO₄ filtered and the solvent evaporated in vacuo giving a resin.

### (6RS,9RS)-(±)-2-Diethylamino-N-(1-dimethylamino-6,9-diphenyl-3-azabicyclo[3.2.2]non-3-yl)acetamide

To the (6RS,9RS)-(±)-2-chloro-N-(1-dimethylamino-6,9-diphenyl-3-azabicyclo[3.2.2]non-3-yl)acetamide a solution of diethylamine in ethanol and a catalytic amount of KI is added. The solution is stirred over night at room temperature under an atmosphere of Ar. Benzene is added to the solution and the solvents are evaporated. The residue is dissolved in CH₂Cl₂ and shaken with 1N NaOH. The organic phase is separated and washed with water until the water phase reacts neutral. Then the organic phase is dried (Na₂SO₄), filtered and the solvent evaporated in vacuo giving a resin.

### (6RS,9RS)-(±)-3-(2-Diethylaminoethyl)-1-dimethylamino-6,9-diphenyl-3-azabicyclo[3.2.2]nonane

The (6RS,9RS)-(±)-2-diethylamino-N-(1-dimethylamino-6,9-diphenyl-3-azabicyclo[3.2.2]non-3-yl)acetamide is suspended in dry ether with stirring and cooling. Powdered LiAlH₄ is added in portions. The suspension is refluxed for two days, cooled to room temperature and quenched cautiously with water under stirring and cooling on an ice bath. 2N NaOH is added and the mixture is extracted with ether, the combined organic phases are washed twice with water, dried (Na₂SO₄ filtered and the solvent evaporated giving a resin.

### Example 31:

### (6RS,9RS)-(±)-3-(2-Diethylaminoethyl)-6,9-diphenyl-1-pyrrohdino-3-azabicyclo[3.2.2]nonane

### (6RS,9RS)-(±)-2-Chloro-N-(6,9-diphenyl-1-pyrrolidino-3-azabicyclo[3.2.2]non-3-yl)acetamide

(6RS,9RS)-(±)-6,9-Diphenyl-1-pyrrolidino-3-azabicyclo[3.2.2]nonane gives with chloroacetyl chloride in dry CH₂Cl₂ in the presence of triethylamine a resin.

### (6RS,9RS)-(±)-2-Diethylamino-N-(6,9-diphenyl-1-pyrrolidino-3-azabicyclo[3.2.2]non-3-yl)acetamide

(6RS,9RS)-(±)-2-Chloro-N-(6,9-diphenyl-1-pyrrolidino-3-azabicyclo[3.2.2]non-3-yl)acetamide gives with diethylamine in the presence of KI a resin.

### (6RS,9RS)-(±)-3-(2-Diethylaminoethyl)-6,9-diphenyl-1-pyrrolidino-3-azabicyclo[3.2.2]nonane

(6RS,9RS)-(±)-2-Diethylamino-N-(6,9-diphenyl-1-pyrrolidino-3-azabicyclo[3.2.2]non-3-yl)acetamide gives with LiAlH₄ in dry ether a resin.

### Example 32:

### (6RS,9RS)-(+)-3-(2-Diethylaminoethyl)-6,9-diphenyl-1-piperidino-3-azabicyclo[3.2.2]nonane

### (6RS,9RS)-(±)-2-Chloro-N-(6,9-diphenyl-1-piperidino-3-azabicyclo[3.2.2]non-3-yl)acetamide

(6RS,9RS)-(±)-6,9-Diphenyl-1-piperidino-3-azabicyclo[3.2.2]nonane gives with chloroacetyl chloride in dry CH₂Cl₂ in the presence of triethylamine a resin.

### (6RS,9RS)-(±)-2-Diethylamino-N-(6,9-diphenyl-1-piperidino-3-azabicyclo[3.2.2]non-3-yl)acetamide

(6RS,9RS)-(±)-2-Chloro-N-(6,9-diphenyl-1-piperidino-3-azabicyclo[3.2.2]non-3-yl)acetamide gives with diethylamine in the presence of KI a resin.

### (6RS,9RS)-(±)-3-(2-Diethylaminoethyl)-6,9-diphenyl-1-piperidino-3-azabicyclo[3.2.2]nonane

(6RS,9RS)-(±)-2-Diethylamino-N-(6,9-diphenyl-1-piperidino-3-azabicyclo[3.2.2]non-3-yl)acetamide gives with LiAlH₄ in dry ether a resin.

### Example 33:

### 5-Phenyl-1-pyrrolidino-3-azabicyclo[3.2.2]nonane

### 1-Phenyl-4-pyrrolidinobicyclo[2.2.2]octan-2-one

3.39 g (15.67 mmol) of 4-Acetyl-4-phenyl-cyclohexanone are dissolved in75.5 ml of benzene and 1.45 g (20.38 mmol) of pyrrolidine and 66.5 mg (0.05 mmol) of p-toluene-sulfonic acid is added. The mixture is refluxed over night at 120°C over a water separator. The solvent was evaporated and the round bottomed flask containing the residue connected with a Kugelrohr distillation apparatus and heated to 250°C for 15-20 min. Subsequent distillation in vacuo yielded the crude product which was recrystallized from ethyl acetate. Yield: 800 mg (19%) white needles. M.p.:143°C
1H NMR (CDCl₃, δ, 400 MHz): 1.76 - 1.82 (m, 4H), 1.88 (ddd, J = 10.9, 10.7, 5.1 Hz, 2H), 1.97 (ddd, J = 12.6, 10.6, 4.2 Hz, 2H), 2.10 (ddd, J = 13.3, 10.8, 5.0 Hz, 2H), 2.25 (ddd, J = 11.0, 10.7, 4.5 Hz, 2H), 2.54 (s, 2H), 2.64 - 2.72 (m, 4H), 7.18 - 7.36 (m, 5H) ppm.

### 1-Phenyl-5-pyrrolidino-3-azabicyclo[3.2.2]nonan-2-one

The hydrochloride of 1-phenyl-4-pyrrolidinobicyclo[2.2.2]octan-2-one gives with NaN₃ in concentrated sulfuric acid a resin.

### 5-Phenyl-1-pyrrolidino-3-azabicyclo[3.2.2]nonane

1-Phenyl-5-pyrrolidino-3-azabicyclo[3.2.2]nonan-2-one gives with LiAlH₄ in dry ether a resin.

### Example A:

3-Azabicyclo[3.2.2.]nonane derivatives of the general formula I or their pharmaceutical applicable salts can be used for the production of pharmaceuticals using known methods.
1. Tablets 500 mg

| | |
|---|---|
| Active ingredient | 500 mg |
| Lactose | 149 mg |
| Polyvinylpyrrolidone | 15 mg |
| Dioctylsodium sulfosuccinate | 1 mg |
| Na-Carboxymethylstarch | 30 mg |
| Magnesium stearate | 5 mg |
| | 700 mg |

2. Tablets 50 mg

| | |
|---|---|
| Active ingredient | 50 mg |
| Lactose | 50 mg |
| Microcrystalline Cellulose | 82 mg |
| Na-Carboxymethylstarch | 18 mg |
| | 200 mg |

3. Capsules 100 mg

| | |
|---|---|
| Active ingredient | 100.0 mg |
| Lactose | 104.7 mg |
| Corn starch | 70.0 mg |
| Dioctylsodium sulfosuccinate | 0.3 mg |
| Magnesium stearate | 3.0 mg |
| Talcum | 12.0 mg |
| Hydroxypropylmethylcellulose | 10.0 mg |
| | 300.0 mg |

4. Suppositories 500 me

| | |
|---|---|
| Active ingredient | 500 mg |
| Suppository mass | 1500 mg |
| | 2000 mg |

5. gelatine capsules 100 mg

| | |
|---|---|
| Active ingredient | 100 mg |
| Medium-chained triglycerides | 300 mg |
| | 400 mg |

## Claims

1. A compound of formula (I) 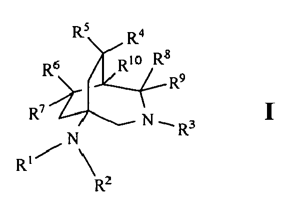 or a pharmaceutically acceptable salt thereof
wherein
R¹ and R² each independently are
(1) hydrogen
(2) C₁-C₁₀alkyl, optionally substituted with a substituent containing a nitrogen atom or with an optionally substituted 3 to 7 membered non-aromatic heterocyclic ring,
(3) an acyl group selected from
an optionally substituted C₁-C₅alkylcarbonyl,
an optionally substituted C₅-C₁₄arylcarbonyl,
an optionally substituted C₅-C₁₄aryl C₁-C₅alkylcarbonyl,
an optionally substituted 6 to 10 membered heteroarylcarbonyl, and
an optionally substituted 6 to 10 membered heteroaryl-C₁-C₅alkylcarbonyl,
(4) C₆-C₁₄aryl-C₁-C₄alkyl, or
(5) heteroaryl-C₁-C₄alkyl;
or
R¹ and R², taken together with the nitrogen, form a 3 to 7 membered non-aromatic heterocyclic ring which may be substituted or may contain one or more additional heteroatoms;
R³ is selected as defined above for R¹ and R² according to (1) to (5);
R⁴ is hydrogen, C₁-C₁₀alkyl, optionally substituted C₆-C₁₀aryl or optionally substituted 5 to 10 membered heteroaryl;
R⁵ is hydrogen;
R⁶ is hydrogen, C₁-C₁₀alkyl, optionally substituted C₆-C₁₀aryl or optionally substituted 5 to 10 membered heteroaryl; R⁶ and R⁴ can be identical or can differ from each other;
R⁷ is hydrogen, C₁-C₁₀alkyl, optionally substituted C₆-C₁₀aryl or optionally substituted 5 to 10 membered heteroaryl;
R⁸ and R⁹ are either hydrogen or together a double bonded oxygen; and
R¹⁰ is hydrogen, C₁-C₁₀alkyl being optionally substituted with a substituent containing a nitrogen atom or with an optionally substituted 3 to 7 membered non-aromatic heterocyclic ring, optionally substituted C₆-C₁₄aryl or optionally substituted heteroaryl.

2. A compound of formula (I) according to claim 1, wherein the substituent containing a nitrogen atom is selected from primary, secondary and tertiary amines, and cyano.

3. A compound of formula (I) according to claim 1, wherein R¹ and R², taken together with the nitrogen, form an optionally substituted 3 to 7 membered heterocyclic ring selected from pyrrolidine, piperidine, morpholine, N-methylpiperazine, azepane, aziridine and azetidine, preferably an optionally substituted 5 to 6 membered heterocyclic ring selected from pyrrolidine, piperidine, morpholine and N-methylpiperazine.

4. A compound of formula (I) according to any one of claims 1 to 3, wherein R⁴ and R⁶ each independently represent phenyl being optionally substituted with 1 to 5 substituents selected from the group consisting of halogen, methyl and amino, and R⁷ represents H.

5. A compound according to claim 1 selected from the group consisting of
(6RS,9RS)-(±)-1-Dimethylamino-6,9-diphenyl-3-azabicyclo[3.2.2]nonane
(6RS,9RS)-(±)-1-Morpholino-6,9-diphenyl-3-azabicyclo[3.2.2]nonane
(6RS,9RS)-(±)-6,9-Diphenyl-1-pyrrolidino-3-azabicyclo[3.2.2]nonane
(6RS,9RS)-(±)-6,9-Diphenyl-1-piperidino-3-azabicyclo[3.2.2]nonane
(6RS,9RS)-(±)-1-(4-Methylpiperazin-1-yl)-6,9-diphenyl-3-azabicyclo[3.2.2]nonane
(6RS,9RS)-(±)-6,9-Bis(4-chlorophenyl)-1-dimethylamino-3-azabicyclo[3.2.2]nonane
(6RS,9RS)-(±)-6,9-Bis(4-chlorophenyl)-1-pyrrolidino-3-azabicyclo[3.2.2]nonane
(6RS,9RS)-(±)-6,9-Bis(4-chlorophenyl)-1-piperidino-3-azabicyclo[3.2.2]nonane
(6RS,9RS)-(±)-1-Dimethylamino-6,9-bis(4-methylphenyl)-3-azabicyclo[3.2.2]nonane
(6RS,9RS)-(±)-6,9-Bis(4-methylphenyl)-1-pyrrolidino-3-azabicyclo[3.2.2]nonane
(6RS,9RS)-(±)-6,9-Bis(4-methylphenyl)-1-piperidino-3-azabicyclo[3.2.2]nonane
(6RS,9RS)-(±)-1-Methylamino-6,9-diphenyl-3-azabicyclo[3.2.2]nonane
(6RS,9RS)-(±)-6,9-Bis(2-chlorophenyl)-1-dimethylamino-3-azabicyclo[3.2.2]nonane
(6RS,9RS)-(±)-6,9-Bis(2-chlorophenyl)-1-pyrrolidino-3-azabicyclo[3.2.2]nonane
(6RS,9RS)-(±)-6,9-Bis(2-chlorophenyl)-1-piperidino-3-azabicyclo[3.2.2]nonane
(6RS,9RS)-(±)-1-(N-(2-Diethylaminoethyl)-N-methylamino)-6,9-diphenyl-3-azabicyclo[3.2.2]nonane
(6RS,9RS)-(±)-1-(N-(2-Dipropylaminoethyl)-N-methylamino)-6,9-diphenyl-3-azabicyclo[3.2.2]nonane
(6RS,9RS)-(±)-1-(N-Methyl-N-(2-pyrrolidinoethyl)amino)-6,9-diphenyl-3-azabicyclo[3.2.2]nonane
(6RS,9RS)-(±)-1-(N-Methyl-N-(2-piperidinoethyl)amino)-6,9-diphenyl-3-azabicyclo[3.2.2]nonane
(6RS,9RS)-(±)-1-(N-Methyl-N-(2-(4-methylpiperazin-1-yl)ethyl)amino)-6,9-diphenyl-3-azabicyclo[3.2.2]nonane
(6RS,9RS)-(±)-3-Ethylamino-6,9-diphenyl-1-pyrrolidino-3-azabicyclo[3.2.2]nonane
(6RS,9RS)-(±)-1-(N-Methyl-N-(pyridin-3-ylinethyl)amino)6,9-diphenyl-3-azabicyclo[3.2.2]nonane
(6RS,9RS)-(±)-6-(4-Aminophenyl)-9-phenyl-1-pyrrolidino-3-azabicyclo[3.2.2]nonane
(6RS,9RS)-(±)-1-(N-(3-Aminopropyl)-N-methylamino)-6,9-diphenyl-3-azabicyclo[3.2.2]nonane
5-Phenyl-1-pyrrolidino-3-azabicyclo[3.2.2]nonane

6. A compound according to claim 1 selected from the group consisting of
(7RS,8RS)-(±)-5-Dimethylamino-7,8-diphenyl-3-azabicyclo[3.2.2]nonan-2-one
(7RS,8RS)-(±)-5-Morpholino-7,8-diphenyl-3-azabicyclo[3.2.2]nonan-2-one
(7RS,8RS)-(±)-7,8-Diphenyl-5-pyrrolidino-3-azabicyclo[3.2.2]nonan-2-one
(7RS,8RS)-(±)-7,8-Diphenyl-5-piperidino-3-azabicyclo[3.2.2]nonan-2-one
(7RS,8RS)-(±)-5-(4-Methylpiperazin-1-yl)-7,8-diphenyl-3-azabicyclo[3.2.2]nonan-2-one
(7RS,8RS)-(±)-7,8-Bis(4-chlorophenyl)-5-dimethylamino-3-azabicyclo[3.2.2]nonan-2-one
(7RS,8RS)-(±)-7,8-Bis(4-chlorophenyl)-5-pyrrolidino-3-azabicyclo[3.2.2]nonan-2-one
(7RS,8RS)-(±)-7,8-Bis(4-chlorophenyl)-5-piperidino-3-azabicyclo[3.2.2]nonan-2-one
(7RS,8RS)-(±)-5-Dimethylamino-7,8-bis(4-methylphenyl)-3-azabicyclo[3.2.2]nonan-2-one
(7RS,8RS)-(±)-7,8-Bis(4-methylphenyl)-5-pyrrolidino-3-azabicyclo[3.2.2]nonan-2-one
(7RS,8RS)-(±)-7,8-Bis(4-methylphenyl)-5-piperidino-3-azabicyclo[3.2.2]nonan-2-one
(7RS,8RS)-(±)-5-Methylamino-7,8-diphenyl-3-azabicyclo[3.2.2]nonan-2-one
(7RS,8RS)-(±)-7,8-Bis(2-chlorophenyl)-5-dimethylamino-3-azabicyclo[3.2.2]nonan-2-one
(7RS,8RS)-(±)-7,8-Bis(2-chlorophenyl)-5-pyrrolidino-3-azabicyclo[3.2.2]nonan-2-one
(7RS,8RS)-(±)-7,8-Bis(2-chlorophenyl)-5-piperidino-3-azabicyclo[3.2.2]nonan-2-one
(6RS,9RS)-(±)-2-Diethylamino-N-methyl-N-(4-oxo-6,9-diphenyl-3-azabicyclo[3.2.2]non-1-yl)acetamide
(6RS,9RS)-(±)-2-Di-(n-propylamino)-N-methyl-N-(4-oxo-6,9-diphenyl-3-azabicyclo[3.2.2]non-1-yl)acetamide
(6RS,9RS)-(±)-N-Methyl-N-(4-oxo-6,9-diphenyl-3-azabicyclo[3.2.2]non-1-yl)-2-pyrrolidino-acetamide
(6RS,9RS)-(±)-N-Methyl-N-(4-oxo-6,9-diphenyl-3-azabicyclo[3.2.2]non-1-yl)-2-piperidino-acetamide
(6RS,9RS)-(±)-N-Methyl-2-(4-methylpiperazin-1-yl)-N-(4-oxo-6,9-diphenyl-3-azabicyclo[3.2.2]non-1-yl)acetamide
(6RS,9RS)-(±)-2-Cyano-N-methyl-N-(4-oxo-6,9-diphenyl-3-azabicyclo[3.2.2]non-1-yl)acetamide
(6RS,9RS)-(±)-3-(2-Aminoethyl)-6,9-diphenyl-1-pyrrolidino-3-azabicyclo[3.2.2]nonane
(6RS,9RS)-(±)-3-(2-Diethylaminoethyl)-1-dimethylamino-6,9-diphenyl-3-azabicyclo[3.2.2]nonane
(6RS,9RS)-(±)-3-(2-Diethylan-dnoethyl)-6,9-diphenyl-l-pyrrohdino-3-azabicyclo[3.2.2]nonane
(6RS,9RS)-(±)-3-(2-Diethylaminoethyl)-6,9-diphenyl-1-piperidino-3-azabicyclo[3.2.2]nonane 1-Phenyl-5-pyrrolidino-3-azabicyclo[3.2.2]nonan-2-one

7. A method for producing a compound of formula (I) according to claim 1 in which R¹ and R² each independently represent C₁-C₁₀alkyl being optionally substituted with a substituent containing a nitrogen atom or with an optionally substituted 3-7 membered non-aromatic heterocyclic ring, C₆-C₁₄aryl-C₁-C-₄alkyl, or heteroaryl-C₁-C₄alkyl; or R¹ and R², taken together with the nitrogen, form a 3 to 7 membered non-aromatic heterocyclic ring which may be substituted or may contain one or more additional heteroatoms; R³ = H; R⁴ = R⁶ = aryl or heteroaryl as defined in claim 1; R⁵ = R⁷ = R¹⁰ = H, comprising the steps of
a) reacting compounds of formula: 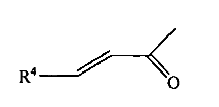 with a compound of formula: 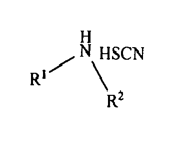 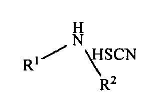 in a boiling organic solvent with water separation or without solvent to obtain a compound of formula (**IV**): 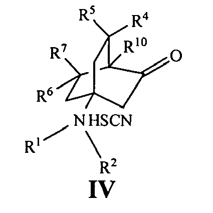
b) isomerisation of a free base of the compound of the formula (**IV**) in the presence of an alkaline catalyst under heating with or without solvent to obtain a compound of formula (**V**): 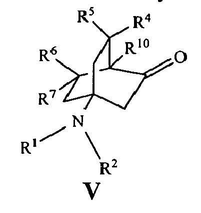
c) a Schmidt-reaction by dissolving the compound of formula (**V**) in cold concentrated sulphuric acid and by treatment with sodium azide to obtain a compound of formula (**VI**): 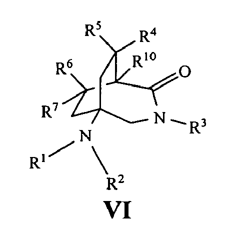
d) and reduction of the compound of formula (**VI**) to a compound of formula (**I**).
in a boiling organic solvent with water separation or without solvent to obtain a compound of formula (**III**): 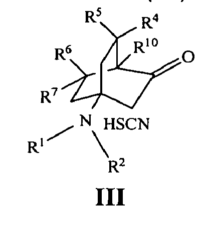
b) a Schmidt-reaction by dissolving the compound of formula (**III**) in cold concentrated sulphuric acid and by treatment with sodium azide to obtain a compound of formula (**II**): 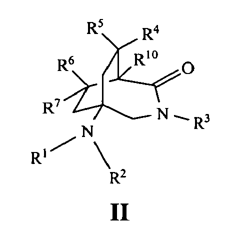
c) and of the compound of formula (**II**) to a compound of formula (**I**).

8. A method for producing a compound of formula (**I**) according to claim 1 in which R¹ and R² each independently represent C₁-C₁₀alkyl being optionally substituted with a substituent containing a nitrogen atom or with an optionally substituted 3-7 membered non-aromatic heterocyclic ring, C₆-C₁₄aryl-C₁-C₄alkyl, or heteroaryl-C₁-C₄alkyl; or R¹ and R², taken together with the nitrogen, form a 3 to 7 membered non-aromatic heterocyclic ring which may be substituted or may contain one or more additional heteroatoms; R³ = H; R⁴ = R⁷ = aryl or heteroaryl as defined in claim 1; R⁵ = R⁶ = R¹⁰ = H, comprising the steps of
a) reacting compounds of formula: 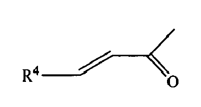 with a compound of formula:

9. A method for producing a compound of formula (**I**) according to claim 1 in which R¹ and R² each independently represent C₁-C₁₀alkyl being optionally substituted with a substituent containing a nitrogen atom or with an optionally substituted 3-7 membered non-aromatic heterocyclic ring, C₆-C₁₄aryl-C₁-C₄alkyl, or heteroaryl-C₁-C₄alkyl; or R¹ and R², taken together with the nitrogen, form a 3 to 7 membered non-aromatic heterocyclic ring which may be substituted or may contain one or more additional heteroatoms; R³ = H; R⁴ ≠ R⁶ = H, aryl or heteroaryl as defined in claim 1; R⁵ = R⁷ = H, R¹⁰ = hydrogen, C₁-C₁₀alkyl being optionally substituted with a substituent containing a nitrogen atom or with an optionally substituted 3 to 7 membered non-aromatic heterocyclic ring, optionally substituted C₆-C₁₄aryl or optionally substituted heteroaryl; comprising the steps of
a(i)) reacting a compound of the following formula, wherein R is alkyl: 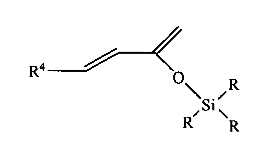 with a compound of formula: 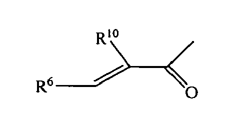 via a Diels-Alder procedure to obtain a compound of formula (**VII**): 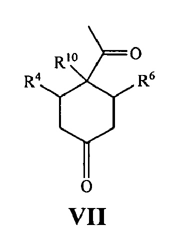 or
a(ii)) providing a pimelic acid derivative of the following formula, wherein R is H or alkyl: 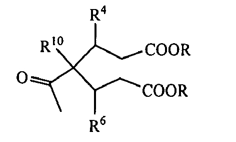 and cyclization of the pimelic acid derivative to obtain a compound of formula (**VIIa**): 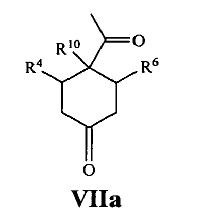
b) reaction of the compound of formula (**VII**) or of formula (**VIIa**) with secondary amines to obtain a compound of formula (**VIII**): 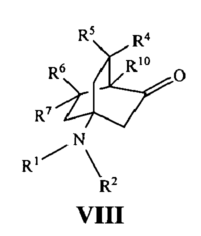
c) a Schmidt-reaction by dissolving the compound of formula (**VIII**) in cold concentrated sulphuric acid and by treatment with sodium azide to obtain a compound of formula (**IX**): 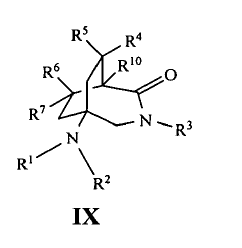
d) and reduction of the compound of formula (**IX**) to a compound of formula (**I**).

10. A method for producing a compound of formula (I) according to claim 1 in which R¹ and R² each independently represent hydrogen, C₁-C₁₀alkyl being optionally substituted with a substituent containing a nitrogen atom or with an optionally substituted 3-7 membered non-aromatic heterocyclic ring, an acyl group selected from an optionally substituted C₁-C₅alkylcarbonyl, an optionally substituted C₅-C₁₄arylcarbonyl, an optionally substituted C₅-C₁₄aryl C₁-C₅alkylcarbonyl, an optionally substituted 6 to 10 membered heteroarylcarbonyl, and an optionally substituted 6 to 10 membered heteroaryl-C₁-C₅alkylcarbonyl, C₆-C₁₄aryl-C₁-C₄alkyl, or heteroaryl-C₁-C₄alkyl; or R¹ and R², taken together with the nitrogen, form a 3 to 7 membered non-aromatic heterocyclic ring which may be substituted or may contain one or more additional heteroatoms; R³ = H; and R⁴, R⁵, R⁶, R⁷, R⁸, R⁹ and R¹⁰ are as defined in claim 1, comprising the steps of
a) oxidation of the C₁-C₁₀alkyl group of either R¹ or R² of free bases of a compound of formula (**III**), (**IV**), (**V**) or (**VIII**) as produced according to any one of claims 7, 8 or 9: 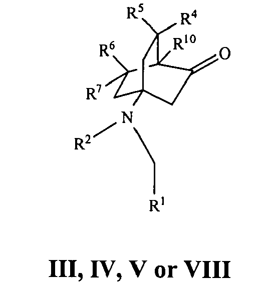 to obtain a compound of formula (**X**): 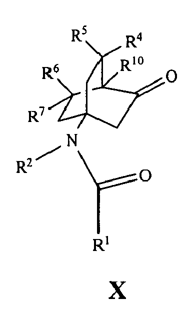
b) hydrolysis of the compound of formula (**X**) to obtain a compound of formula (**XI**): 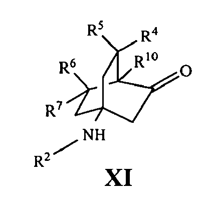
c) N-alkylation or N-acylation of the compound of formula (**XI**) to obtain a compound of formula (**XII**): 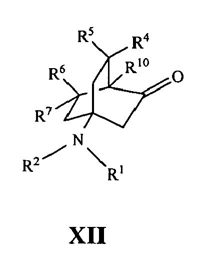
d) and a Schmidt-reacdon by dissolving the compound of formula (XII) in cold concentrated sulphuric acid and by treatment with sodium azide followed by a reduction step to obtain a compound of formula (**I**).

11. A compound according to any one of claims 1 to 6 as a therapeutically active substance.

12. A compound according to any one of claims 1 to 6 as a therapeutically active substance for treating or preventing a disease or condition associated with strains selected from chloroquine-resistant or chloroquine-sensitive strains of the causative organisms of malaria and the causative organism of sleeping sickness.

13. A pharmaceutical composition comprising at least one compound according to any one of claims 1 to 6 or a pharmaceutically acceptable salt thereof as active ingredient.

14. A pharmaceutical composition comprising as an active incredient at least one compound according to any one of claims 1 to 6 or a pharmaceutically acceptable salt thereof and at least one of a pharmaceutically acceptable carrier and/or excipient.

15. A pharmaceutical composition for treating or preventing a disorder or condition selected from malaria and sleeping sickness, comprising an amount of a compound according to any one of claims 1 to 6, or a pharmaceutically acceptable salt thereof, that is effective in treating such disorder or condition and a pharmaceutically acceptable carrier and/or excipient.

16. Use of a compound according to any one of claims 1 to 6 for the manufacture of a medicament.

17. The use according to claim 16, wherein the medicament is for treating or preventing a disease or condition associated with strains selected from chloroquine-resistant or chloroquine-sensitive strains of the causative organisms of malaria and the causative organism of sleeping sickness.
